(19) Europäisches Patentamt
European Patent Office
Office européen des brevets

(11) EP 2 295 032 A1

# (12) EUROPEAN PATENT APPLICATION

published in accordance with Art. 153(4) EPC

(43) Date of publication:
**16.03.2011 Bulletin 2011/11**

(21) Application number: **09754807.7**

(22) Date of filing: **29.05.2009**

(51) Int Cl.:
***A61K 8/68*** (2006.01) ***A23L 1/30*** (2006.01)
***A61K 8/06*** (2006.01) ***A61K 8/86*** (2006.01)
***A61K 31/164*** (2006.01) ***A61Q 19/00*** (2006.01)
***B01J 13/00*** (2006.01) ***A61P 17/16*** (2006.01)

(86) International application number:
**PCT/JP2009/059865**

(87) International publication number:
**WO 2009/145299 (03.12.2009 Gazette 2009/49)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **29.05.2008 JP 2008141181**

(71) Applicant: **FUJIFILM Corporation**
**Tokyo 106-8620 (JP)**

(72) Inventors:
- **SERIZAWA, Shinichiro**
  **Ashigarakami-gun**
  **Kanagawa 258-8577 (JP)**
- **MORI, Hisahiro**
  **Ashigarakami-gun**
  **Kanagawa 258-8577 (JP)**
- **ARAKAWA, Jun**
  **Ashigarakami-gun**
  **Kanagawa 258-8577 (JP)**
- **TASHIRO, Tomoko**
  **Ashigarakami-gun**
  **Kanagawa 258-8577 (JP)**
- **KUBO, Toshiaki**
  **Ashigarakami-gun**
  **Kanagawa 258-8577 (JP)**
- **NAKAMURA, Yoshisada**
  **Ashigarakami-gun**
  **Kanagawa 258-8577 (JP)**
- **UEYAMA, Tomohide**
  **Ashigarakami-gun**
  **Kanagawa 258-8577 (JP)**

(74) Representative: **HOFFMANN EITLE**
**Patent- und Rechtsanwälte**
**Arabellastraße 4**
**81925 München (DE)**

## (54) CERAMIDE DISPERSION

(57) A ceramide dispersion, which includes at least: natural-ceramide-containing particles that are dispersed in an aqueous phase as an oil-phase component; and at least one surfactant, wherein the natural-ceramide-containing particles contain one or more natural ceramides in an amount of 50% by mass or higher with respect to the total mass of the oil components contained in an oil phase, and have a volume average particle diameter of from 0.5 nm to 100 nm; and the ceramide dispersion contains, in the at least one surfactant, at least one polyglycerin fatty acid ester having an HLB of from 10 to 16.



Printed by Jouve, 75001 PARIS (FR)

## Description

### TECHNICAL FIELD

The present invention relates to a ceramide dispersion, and, in more detail, to a ceramide dispersion containing natural-ceramide-containing particles having small particle diameters.

### BACKGROUND ART

Ceramides are present in keratin layers of skin, form a lipid barrier that is required to preserve moisture, and play an important role for maintaining moisture levels. Ceramides present in keratin layers are a substance generated by decomposition of cerebroside by an enzyme called cerebrosidase. It is well known that a part of ceramide is changed into phytosphingosine and sphingosine by an enzyme called ceramidase and is an important regulator for expansion and differentiation of cells. Human skin contains six different kinds of ceramides, each of which has a different function. However, a ceramide is a highly crystalline substance and has a low solubility in other oil solutions so that it precipitates crystals at a low temperature. Therefore, when ceramides are incorporated into a cosmetic material, it is difficult to secure stability. In addition, an aqueous ceramide dispersion can be dispersed by using, for example, a surfactant, but it is difficult to decrease its particle diameter sufficiently, so that the dispersion tends to lack transparency.

As a composition containing a ceramide, an emulsified composition containing a specific sphingoglycolipid that has a moisturizing effect, a skin chapping prevention effect and an emulsifying effect is disclosed (for example, refer to Japanese Patent Application Laid-Open (JP-A) No. 2000-51676).

In addition, additives for ceramide-mixed cosmetic products containing cholesterol, a fatty acid, and a water-soluble polymer (for example, refer to JP-A No. 7-187987) or a water-in-oil emulsified composition in which a salt formed with a sphingosine and a specific fatty acid is used as an emulsifier and an oil-soluble antioxidant is added in a specific ratio, which is a composition for external application having excellent stability even in the case of abrupt temperature change and also having a good feeling in use, (for example, refer to JP-A No. 2006-335692) are disclosed.

Furthermore, as a formulation technology, a method for manufacturing an additive for a cosmetic material in which a process that makes a coarse dispersion liquid of sphingoglycolipid into fine particles by using a predetermined jet stream is performed to sufficiently develop the emollient effect of the sphingoglycolipid is disclosed (for example, JP-A No. 11-310512).

In addition, as a technique to dissolve a ceramide to yield a transparent solution and mix it stably, mixing a specific fatty acid or a specific surfactant is disclosed (for example, refer to JP-A No. 2001-139796 and JP-A No. 2001-316217). However, to dissolve ceramides to yield transparent solutions, it is necessary to mix a large amount of surfactant, so that there have been cases in which safety or feeling in use is adversely affected. Meanwhile, in a case in which the mixing amount of a surfactant is decreased for obtaining an excellent feeling in use, ceramides often exhibit white turbidity or produce a translucent emulsion, and cannot be fully dissolved in a transparent manner, and, in this case, as time passes, segregation or creaming occurs, so that it has been difficult to secure sufficient stability over time. As such, the above technologies cannot provide a ceramide dispersion in which a ceramide is stably dispersed, and thus cannot fully satisfy the recent strong demand for an emollient effect.

### DISCLOSURE OF INVENTION

### TECHNICAL PROBLEMS

An object of the present invention is to provide a ceramide dispersion in which natural-ceramide-containing particles having small particle diameters are uniformly and stably dispersed.

### SOLUTION TO PROBLEM

Specific means for solving the above problems are as follows:

<1> A ceramide dispersion comprising at least:

natural-ceramide-containing particles that are dispersed in an aqueous phase as an oil-phase component; and at least one surfactant,
wherein
the natural-ceramide-containing particles contain one or more natural ceramides in an amount of 50% by mass or higher with respect to the total mass of oil components contained in an oil phase, and have a volume average

particle diameter of from 0.5 nm to 100 nm; and
the ceramide dispersion contains, in the at least one surfactant, at least one polyglycerin fatty acid ester having an HLB of from 10 to 16.

<2> The ceramide dispersion according to <1>, wherein the at least one surfactant is contained in a range of from 0.1 parts by mass to 2 parts by mass with respect to the total mass of the one or more natural ceramides.
<3> The ceramide dispersion according to <1> or <2>, wherein the polyglycerin fatty acid ester having an HLB of from 10 to 16 is a decaglycerin fatty acid ester.
<4> The ceramide dispersion according to <3>, wherein the decaglycerin fatty acid ester is decaglycerin oleate.
<5> The ceramide dispersion according to <1> or <2>, containing, in the at least one surfactant, decaglycerin oleate and a polyglycerin fatty acid ester having a polymerization degree of glycerin of less than 10 and a carbon number of the fatty acid of from 12 to 18.
<6> The ceramide dispersion according to <5>, wherein the polyglycerin fatty acid ester having a polymerization degree of the glycerin of less than 10 and a carbon number of the fatty acid of from 12 to 18 is at least one selected from a hexaglycerin fatty acid ester or a tetraglycerin fatty acid ester and has an HLB of from 5.0 to 15.
<7> The ceramide dispersion according to any one of <1> to <6>, wherein the one or more natural ceramides do not include sphingoglycolipid and each have three or more hydroxyl groups in a molecular structure thereof.
<8> A cosmetic material comprising the ceramide dispersion according to any one of <1> to <7>.
<9> A food product comprising the ceramide dispersion according to any one of <1> to <7>.
<10> A pharmaceutical product comprising the ceramide dispersion according to any one of <1> to <7>.

## ADVANTAGE EFFECTS OF INVENTION

According to the present invention, it is possible to provide a ceramide dispersion in which natural-ceramide-containing particles having small particle diameters are uniformly and stably dispersed.

## BEST MODE FOR CARRYING OUT THE INVENTION

### [Ceramide Dispersion]

The ceramide dispersion according to the present invention contains at least natural-ceramide-containing particles that are dispersed in an aqueous phase as an oil-phase component and at least one surfactant, wherein the natural-ceramide-containing particles contain one or more natural ceramides in an amount of 50% by mass or higher with respect to the total mass of oil components contained in an oil phase, and have a volume average particle diameter of from 0.5 nm to 100 nm, and the ceramide dispersions contains, in the at least one surfactant, at least one polyglycerin fatty acid ester having an HLB of from 10 to 16.
In the ceramide dispersion according to the present invention, the natural-ceramide-containing particles having small particle diameters are dispersed as an oil-phase component in an aqueous phase uniformly and stably. Therefore, the ceramide dispersion according to the present invention is distinctively characterized by having a high transparency and maintaining the transparency even in the case of containing the natural ceramides at a high concentration.
The ceramide dispersion according to the present invention containing such natural-ceramide-containing particles may take a form of an emulsion having dispersion particles that include, other than the natural ceramides and the surfactant, for example, an oil component or a solvent other than the natural ceramides as the oil component, according to necessity.
In the case of using an oil component and a solvent other than the natural ceramides, the oil component and the solvent are preferably contained at 1 part by mass or less with respect to 1 part by mass of natural-ceramide-containing particles, and more preferably 0.5 parts by mass or less.
In addition, it is possible to include, other than water, a higher alcohol, a fatty acid, a fatty acid salt and/or a compound that can bond with a ceramide by hydrogen bonding in the aqueous phase that serves as a dispersion medium in which the natural-ceramide-containing particles are dispersed. Furthermore, as the aqueous phase, it is possible to use an aqueous solution containing water as the main component. It is also possible to further add a water-soluble functional component, such as a water-soluble antioxidant agent and a botanical extract liquid to the aqueous phase as long as such addition does not adversely affect the effects of the present invention.
Hereinafter, a variety of components contained in the ceramide dispersion according to the present invention are sequentially described.

[Natural Ceramide-containing Particles]

The natural-ceramide-containing particles contained in the ceramide dispersion according to the present invention are particles dispersed in the aqueous phase as an oil-phase component, and contain one or more natural ceramides in an amount of 50% by mass or higher with respect to the total mass of oil components contained in an oil phase, and have a volume average particle diameter of from 0.5 nm to 100 nm.
That is, the ceramide dispersion according to the present invention is characterized by containing the natural ceramides in the form of particles containing the natural ceramides in the dispersion. Such natural-ceramide-containing particles may form an oil phase by being dispersed as dispersion particles containing only the natural ceramides in the aqueous phase as they are, or may form an oil phase together with oil component(s) different from the natural ceramides by being dispersed as dispersion particles containing the natural ceramides and the oil component(s) different from the natural ceramides in the aqueous phase. Meanwhile, a method for forming the natural-ceramide-containing particles will be described below.

(Natural Ceramides)

In the present invention, a natural ceramide refers to a ceramide having the same structure as the ceramide that is present in human skin. More preferable embodiments of the natural ceramide do not include sphingoglycolipid and are those each having three or more hydroxyl groups in a molecular structure thereof.
Hereinafter, the natural ceramides usable for the present invention will be described in detail.
Examples of basic structural formulas of the natural ceramides which may be preferably used in the present invention will be shown in the following (1-1) to (1-10).

(1-1) represents a compound known as Ceramide 1; (1-2) represents a compound known as ceramide 9; (1-3) represents a compound known as ceramide 4; (1-4) represents a compound known as ceramide 2; (1-5) represents a compound known as ceramide 3; (1-6) represents a compound known as ceramide 5; (1-7) represents a compound known as ceramide 6: (1-8) represents a compound known as ceramide 7; (1-9) represents a compound known as ceramide 8; and (1-10) represents a compound known as ceramide 3B.

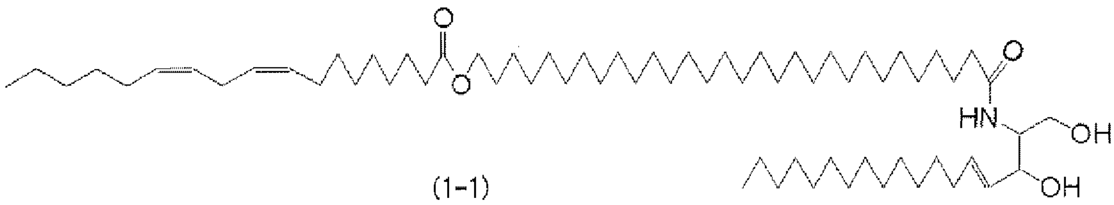

(1-1)

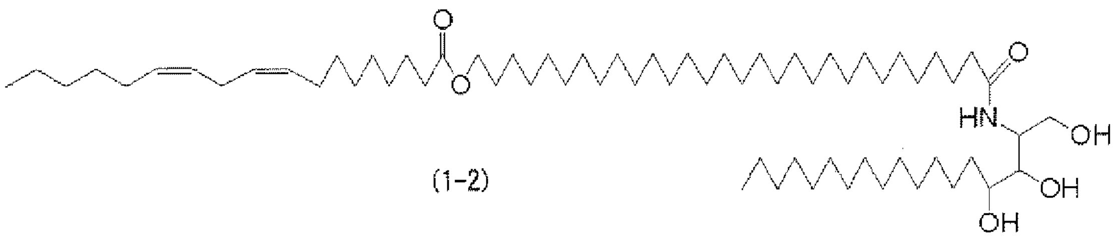

(1-2)

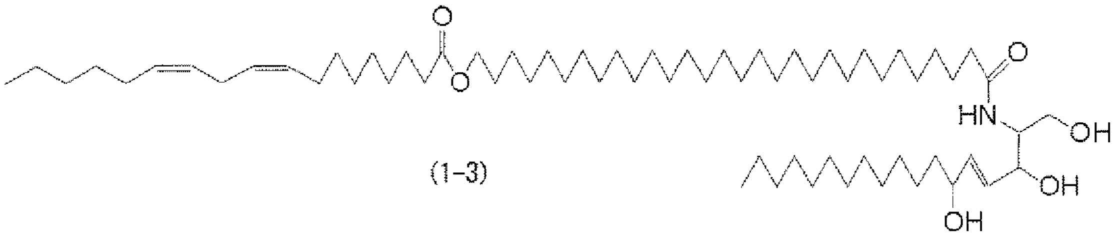

(1-3)

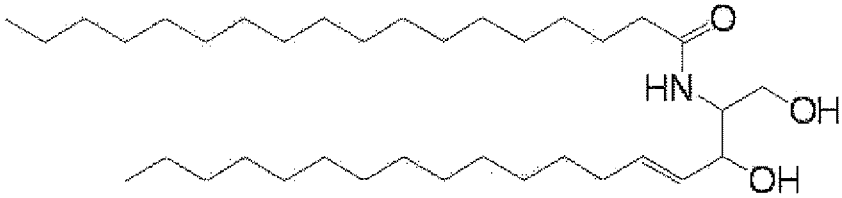

(1-4)

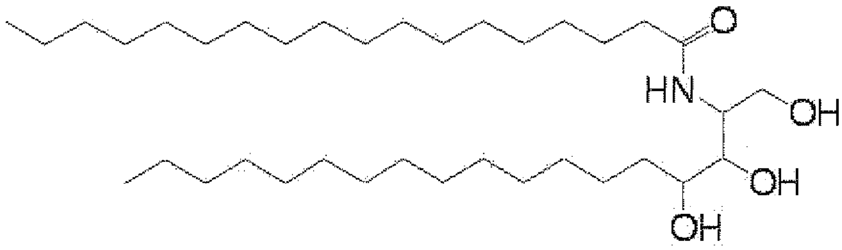

(1-5)

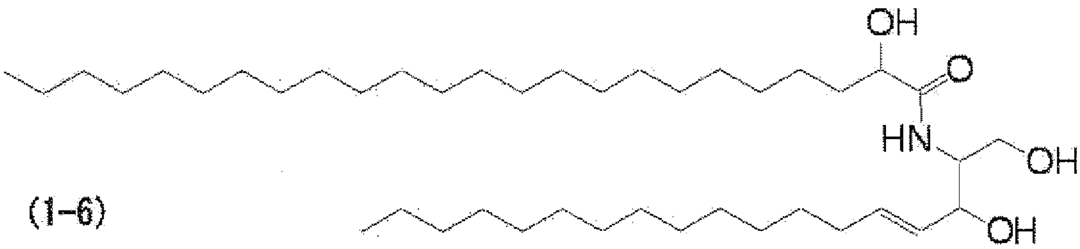

(1-6)

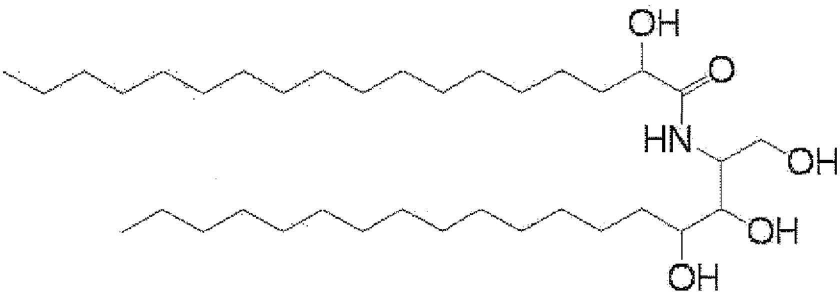

(1-7)

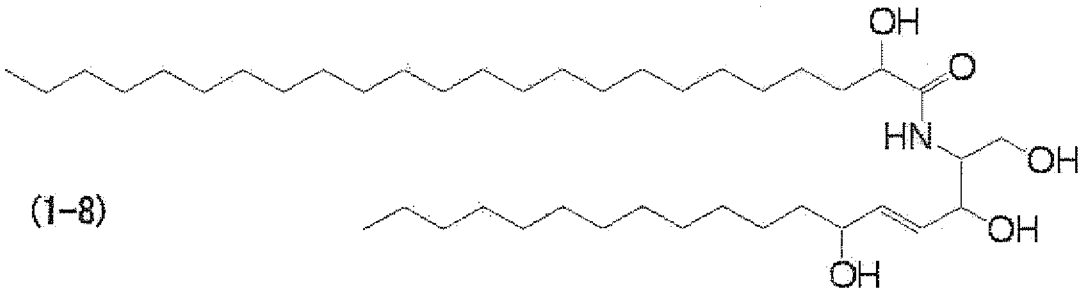

(1-8)

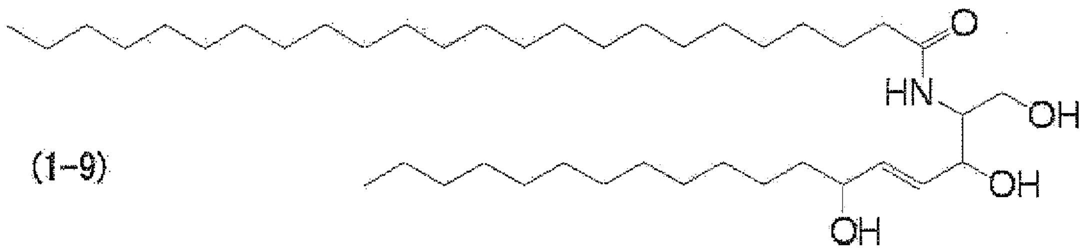

(1-9)

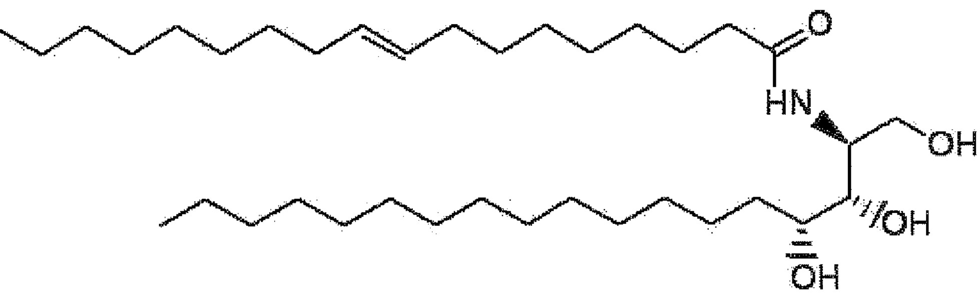

(1-10)

The structural formulas show examples of the respective ceramides, but, since ceramides are natural products, there are a variety of modification examples with respect to the length of the alkyl chains in ceramides that are actually derived from, for example, human beings or animals, therefore the length of the alkyl chains may have any structure as long as they have the above skeletons.
In addition, it is possible to use ceramides that have been modified according to purpose by, for example, introducing double bond(s) in the molecule to provide solubility for the purpose of, for example, incorporating into a formulation, or introducing hydrophobic group(s) to provide permeability.
Examples of these ceramides which are referred as natural ceramides and have common structures include natural products (extracts) and substances obtained by the microbial fermentation method, but may further include synthetic substances and animal-derived ceramides.
The ceramide may be used as a natural (D (-) type) optical isomer. Further, according to necessity, a non-natural (L (+) type) optical isomer may be used. A mixture of a natural optical isomer and a non-natural optical isomer may also be used. The relative configuration of the above compound may be a configuration of a natural type, a configuration of a non-natural type other than the former, or a mixture thereof.
Meanwhile, in the case of using a nanoceramide dispersion or composition for the purpose of, for example, a skin emollient, from the viewpoints of a barrier effect, it is preferable to use more of a natural optical isomer.
These natural ceramides can be obtained as commercially available products, and examples of the commercially

available product include CERAMIDE I, CERAMIDE III, CERAMIDE IIIA, CERAMIDE IIIB, CERAMIDE IIIC, CERAMIDE VI. (manufactured by Cosmoferm), CERAMIDE TIC-001 (manufactured by Takasago International Corporation), CERAMIDE II (manufactured by Quest International Inc.), DS-CERAMIDE VI, DS-CLA-PHYTOCERAMIDE, C6-PHYTOCERAMIDE, DS-CERAMIDE Y3S (manufactured by Doosan Corporation), and CERAMIDE 2 (manufactured by Sederma). In addition, the exemplary compound (1-5) can be obtained as "CERAMIDE 3" [manufactured by Evonik (former Degussa)], and the exemplary compound (1-7) can be obtained as "CERAMIDE 6" (trade name, manufactured by Evonik (former Degussa)).

As for the natural ceramides contained in the natural-ceramide-containing particles, it is possible to use one kind or use two or more kinds in combination, but, normally, ceramides have a high melting point and are highly crystalline and, therefore, it is preferable to use two or more kinds in combination from the viewpoints of emulsion stability and a handling property.

-Content of Natural Ceramides-

In the ceramide dispersion according to the present invention, it is necessary that the natural-ceramide-containing particles contain the natural ceramides in an amount of 50% by mass or higher with respect to the total mass of oil component contained in an oil phase, and from the viewpoints of the effective transdermal absorption or oral absorption of the ceramide component and the expectation of the development of effects caused by the ceramides when applying the ceramide dispersion in a variety of uses, such as a cosmetic material, a pharmaceutical product, and a food product, the content is preferably from 50% by mass to 100% by mass, and more preferably from 75% by mass to 100% by mass. Here, in the ceramide dispersion according to the present invention, the oil components contained in the oil phase refers, among the components contained in the oil phase, not only to ceramides, such as the natural ceramides in the present invention and ceramide analogs that can be used together with the natural ceramides and are described below, but also to oil components having suitable properties and functions for the application purposes of the ceramide dispersion, such as, a variety of oil components, examples of thereof including those of other oil components described below (for example, lipophilic carotenoids, lipophilic vitamins, ubiquinones, fatty acids, and lipids). However, among the components used for preparing the oil phase, the surfactant and a water-soluble organic solvent are not included in the oil components of the present invention.

Meanwhile, the content of the natural ceramides in the ceramide dispersion is preferably in a range of from 0.01% by mass to 5% by mass, and more preferably in a range of from 0.1% by mass to 3% by mass. The content of the natural ceramides in the ceramide dispersion is preferably in the above ranges from the viewpoints of the feeling a user has when the ceramide dispersion is used in a product for external application, such as a cosmetic product.

The natural-ceramide-containing particles may include, together with the above-mentioned natural ceramides contained as an essential component, ceramide-related compound(s), such as glycolsylated ceramide, a ceramide analog, sphingosine or phytosphingnsine in a range that does not adversely affect the effects of the present invention. Meanwhile, in the following description, there are cases in which the natural ceramides that are the essential component and the ceramide-related compounds that are the optional components are collectively referred to as "a ceramide" or "ceramides").

(Glycosylated Ceramide)

A glycosylated ceramide is a ceramide compound containing a saccharide in the molecule thereof. Examples of saccharides contained in the molecule of the ceramide compound include a monosaccharide, such as glucose or galactose, a disaccharide, such as lactose or maltose, and oligosaccharide and polysaccharide formed by polymerizing any of these monosaccharides or disaccharides by a glycosidic bond. The saccharide may be a sugar derivative in which, in the sugar unit thereof, hydroxyl group(s) have been replaced with another group(s). Examples of such a sugar derivative include glucosamine, glucuronic acids, and N-acetyl glucosamine.

Among the above, from the viewpoints of dispersion stability, saccharides with the number of sugar units of from 1 to 5 are preferable, and, specifically, glucose and lactose are preferable, and glucose is more preferable.

Specific examples of the glycosylated ceramide include, for example, the following substances.

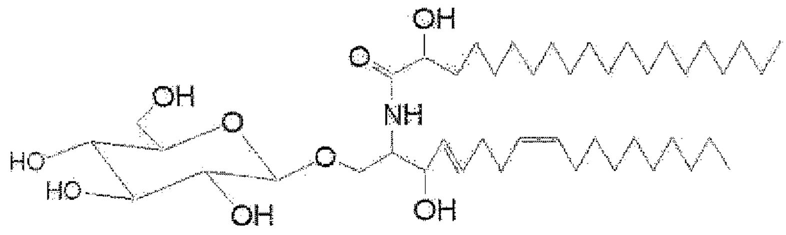

(4-1)

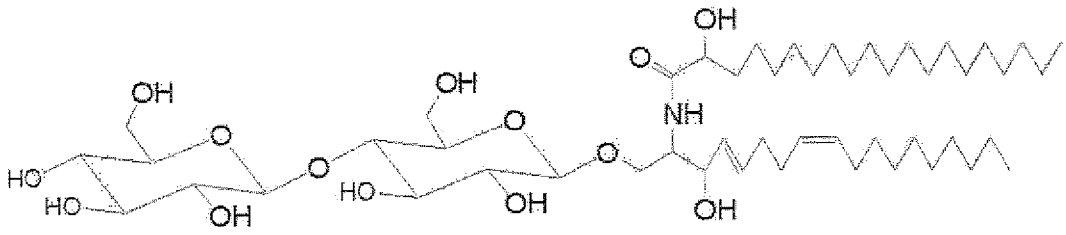

(4-2)

The glycosylated ceramide can be obtained by synthesis or as a commercially available product. For example, the exemplary compound (4-1) can be obtained as "KOME GLYCOSPHIGOLIPID (or RICE GLYCOSPHIGOLIPID) (trade name, manufactured by Okayasu Shoten Co., Ltd.)"

(Ceramide Analog)

A ceramide analog is a substance synthesized to have a similar structure to ceramides. Examples of a well-known compound of such a ceramide analog, which may be used, include a ceramide analog shown in the formula below.
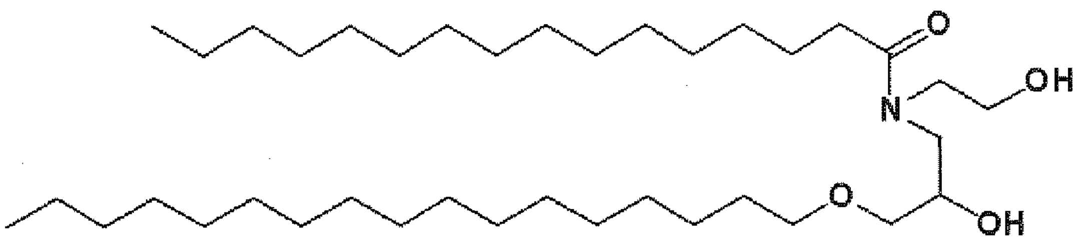

In the case of using the ceramide analogs, for example, when the ceramide dispersion according to the present invention is used as a cosmetic product, from the viewpoints of, such as, feeling in use and moisturized feeling, the analogs of natural ceramides or glycosylated ceramides are preferable, and the analogs of natural ceramides are more preferable.

(Sphingosine, Phytosphingosine)

As the sphingosine and phytosphingosine, it is possible to use any of natural sphingosines and sphingosine analogs, which may be a synthetic product or a natural product, may be used.
Specific examples of the natural sphingosine include sphingosine, dihydrosphingosine, phytosphingosine, sphingadienine, dihydrosphingosine, dihydrophytosphingosine, and the N-alkylated (for example, N-methylated) compounds

thereof and acetylated compounds thereof.

The sphingosine may be used as a natural (D (-) type) optical isomer, a non-natural (L(+) type) optical isomer, or a mixture of a natural optical isomer and a non-natural optical isomer. The relative configuration of the compound may be a configuration of a natural type, a configuration of a non-natural type other than the former, or a mixture thereof. Specific examples include PHYTOSPHINGOSINE (INCI name; 8th Edition) and the following exemplary compounds.

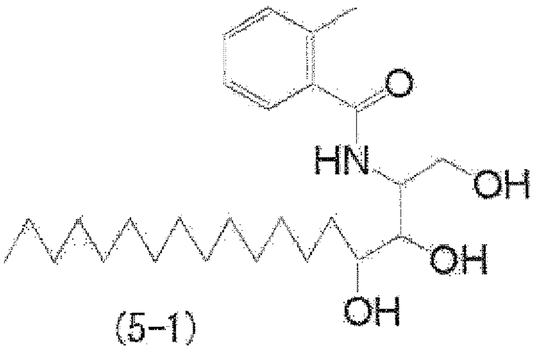

(5-1)

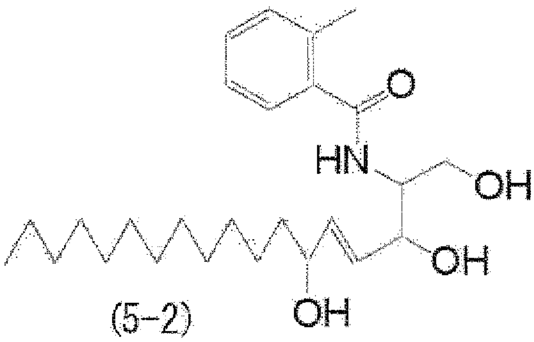

(5-2)

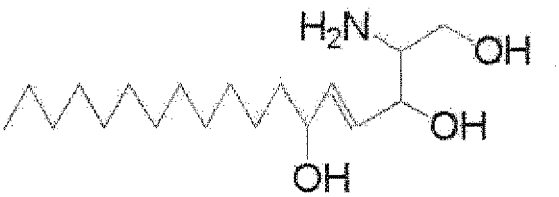

(5-3)

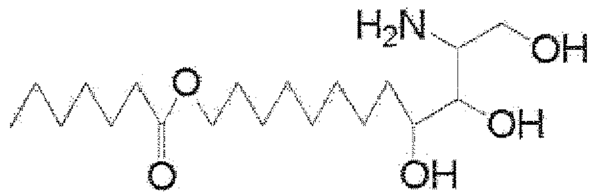

(5-4)

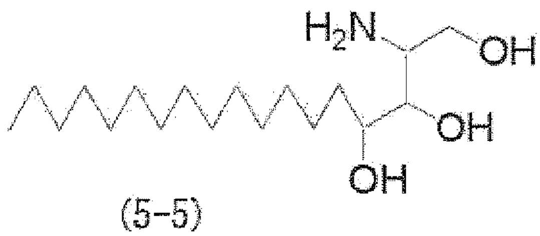

(5-5)

As the phytosphingosine, it is possible to use any of an extract product from a natural substance or a synthetic product. In addition, the phytosphingosine can be obtained by synthesis or as a commercially available product.
Examples of the commercially available products of natural sphingosines include D-SPHINGOSINE (4-Sphingenine) (manufactured by Sigma-Aldrich Co.), DS PHYTOSPHINGOSINE (manufactured by Doosan Corporation), and PHYTOSPHINGOSINE (manufactured by Cosmoferm). The exemplary compound (5-5) can be obtained as "PHYTOSPHINGOSINE (manufactured by Evonik (former Degussa))."

-Acid-

In the present invention, in the case of using a sphingosine compound, such as sphingosine or phytosphingosine, it is preferable to use, in combination with the sphingosine compound, a compound having an acidic residue with which a salt of the compound can be formed. Preferable examples of the compound having an acidic residue include an inorganic acid and an organic acid with a carbon number of 5 or less.
Examples of the inorganic acid include phosphoric acid, hydrochloric acid, nitric acid, sulfuric acid, perchloric acid, and carbonic acid. Phosphoric acid and hydrochloric acid are preferable.
Examples of the organic acid include a monocarboxylic acid, such as a formic acid, acetic acid, propionic acid, butyric acid, isobutyric acid, and valeric acid; a dicarboxylic acid, such as succinic acid, phthalic acid, fumaric acid, oxalic acid, malonic acid, and glutaric acid; an oxycarboxylic 40 acid, such as glycolic acid, citric acid, lactic acid, pyruvic acid, malic acid, and tartaric acid; and an amino acid, such as glutamic acid and asparagine acid. Preferable examples of these organic acid compounds include phosphoric acid, hydrochloric acid, succinic acid, citric acid, lactic acid, glutamic acid, and an asparagine acid, and more preferable examples include lactic acid, glutamic acid, and asparagine acid.
The acid used in combination with the sphingosine compound may be used after being mixed with the sphingosine compound, or may be added when ceramide-related-compoud-containing particles are formed, or may be added as a pH adjuster after ceramide-related-compound-containing particles have been formed.
In the case of using the acid together with the sphingosine compound, the preferable addition amount is from about 1 part by mass to 50 parts by mass with respect to 100 parts by mass of the sphingosine compound(s) used.

-Particle Diameters-

The volume average particle diameter of the natural-ceramide-containing particles is from 0.5 nm to 100 nm, and preferably from 0.5 nm to 75 nm, and more preferably from 0.5 nm to 50 nm, and most preferably from 0.5 nm to 30 nm. By making the particle diameters of the natural-ceramide-containing particles in a range of 0.5 nm to 100 nm, when the

ceramide dispersion according to the present invention is used for for the composition of, such as, a cosmetic product, a pharmaceutical product, and a food product, the transparency of the composition can be ensured and desired effects of, for example, dermal absorption can be satisfactorily developed.

The particle diameters of the natural-ceramide-containing particles can be measured with a commercially available particle size distribution analyzer or the like.

Examples of known methods of measuring the particle diameter distribution include an optical microscopy method, a confocal laser microscopy method, an electron microscopy method, an atomic force microscopy method, a static light scattering method, a laser diffraction method, a dynamic light scattering method, a centrifugal precipitation method, an electric pulse measurement method, a chromatography method, and an ultrasonic damping method, and devices corresponding to the respective principles are commercially available.

When measuring the particles diameters of the natural-ceramide-containing particles in the invention, it is preferable to employ a dynamic light scattering method in consideration of the particle diameter range and ease of measurement.

Examples of commercially available measurement devices using dynamic light scattering include NANOTRAC UPA (Nikkiso Co., Ltd.), a dynamic light scattering particle diameter distribution measuring device LB-550 (Horiba, Ltd.), and a concentrated solution type particle diameter analyzer FPAR-1000 (Otsuka Electronics Co., Ltd.).

The particle diameters of the natural-ceramide-coutaining particles according to the present invention are values measured using a dynamic light scattering particle diameter distribution measuring device LB-550 (Horiba, Ltd.), and, specifically, are measured in the following manner.

That is, a dilution is performed using pure water to make the concentration of the oil components contained in a sample taken from the ceramide dispersion according to the present invention 1% by mass, and then a measurement is performed using a quartz cell. The particle diameters can be obtained as a median size when the refractive index of the sample, the refractive index of the dispersion medium, and the viscosity of the dispersion medium are set to 1.600, 1.333 (pure water), and the viscosity of pure water, respectively.

Examples of an embodiment for containing the natural-ceramide-containing particles in the ceramide dispersion according to the present invention include 1) an embodiment in which the natural-ceramide-containing particles (oil phase) are formed as solid particles in advance, and then dispersed in a dispersion medium (aqueous phase), and 2) an embodiment in which the natural ceramides and ceramide related compounds, which are used according to necessity, are heated to be in a molten state or dissolved in a proper solvent to be in a liquid phase, and then added to and dispersed in an aqueous phase, followed by lowering the temperature to room temperature or removing the solvent, whereby the natural-ceramide-containing particles are formed in the system. In the case of using the 2) embodiment, for example, if the oil phase contains other active ingredients, since there is a concern that the active ingredients may be damaged under a high temperature condition, the temperature of the heating is preferably in a range of 20°C to 60°C. It is preferable to mutually dissolve the natural ceramides and the like and at least one other oil component each other, or to dissolve the natural ceramides and the like in an organic solvent, for the preperation.

As a specific granulation method to make the particle diameters of the natural-ceramide-containing particles small, a well-known method can be applied. In the present invention, a method using a micro mixer in which the oil phase and the aqueous phase independently pass through through a microchannel of which the narrowest portion has a cross-sectional area of from 1 $\mu m^2$ to 1 $mm^2$, and then the oil phase and the aqueous phase are combined and mixed with each other, can be applied. This method is a preferable embodiment of the granulation method of the natural-ceramide-containing particles according to the present invention. The method using a micro mixer is described in detail below. In addition, the natural-ceramide-containing particles may be granulated by using a high-pressure emulsification method in which 100 MPa or more of shearing force is applied or a precipitation method.

[Oil Components Other Than Natural Ceramides]

The ceramide dispersion according to the present invention has a structure in which the natural-ceramide-containing particles are dispersed as an oil phase in an aqueous phase. The ceramide dispersion according to the present invention may take a form in which oil component(s) that are different from the ceramides such as the above-mentioned natural ceramides (in the present specification, such oil components which are other than the ceramides may be referred to as other (or another) oil components) and/or a solvent in the oil phase are contained so that oil droplet-like dispersion particles containing the natural ceramides in the oil components and/or the solvent are present as the natural-ceramide-containing particles. Meanwhile, in the case of taking such a form, the average particle diameter of the natural-ceramide-containing particles according to the present invention refers to the average particle diameter of the oil droplet-like dispersion particles containing the natural-ceramide-containing particles.

Meanwhile, the "other oil components" in the present invention refer to oil components that are not separated from ceramides at room temperature, and the "solvent" refers to a solvent that can dissolve ceramides, and examples thereof includes alcohols.

Here, the other oil components usable in the present invention are not particularly limited. The other oil components

may be components added as active ingredient in accordance with the intended use of the ceramide dispersion, oil components used for improving dispersion stability or feeling with respect to skin or controlling the properties of a composition containing the ceramide dispersion. Hereinbelow, the other oil components usable in the present invention are described.

(Stenone, Sterol)

The ceramide dispersion according to the present invention may contain at least one of stenone and sterol as other oil components. These compounds are useful in improving the dispersion stability of the ceramide dispersion. Specific examples of stenones usable as the other oil components in the present invention include the following.
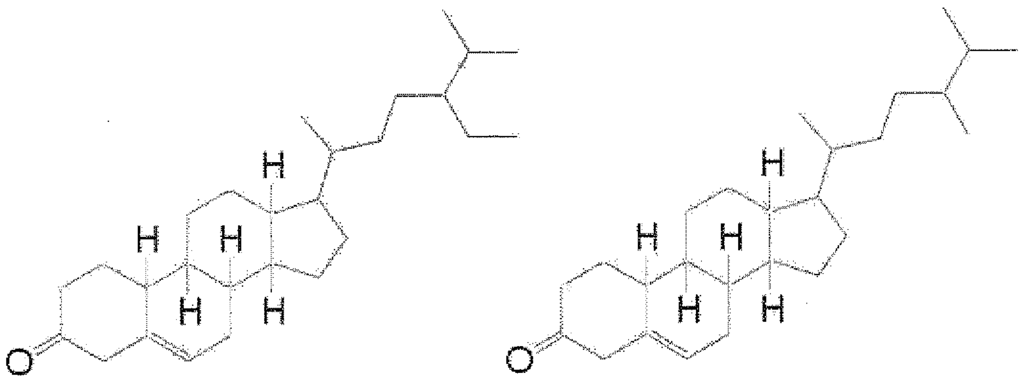

(2-1) (2-2)

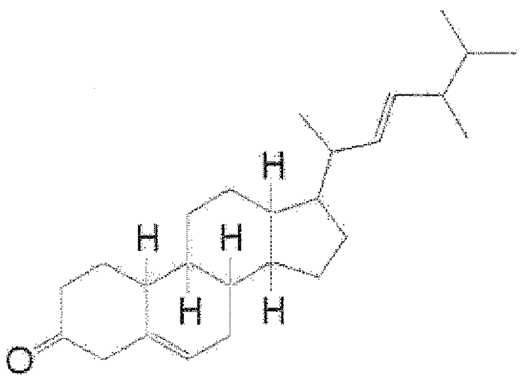

(2-3)

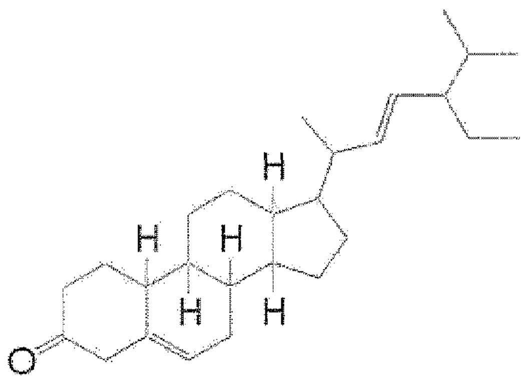

(2-4)

Specific Examples of sterol include the following.
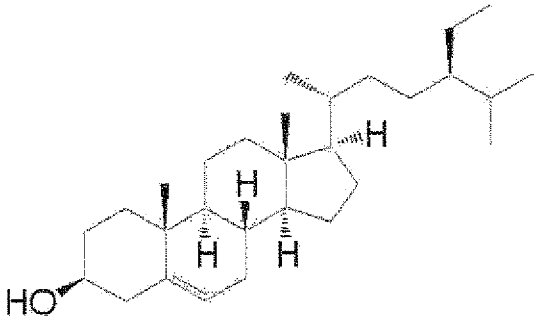

Beta-Sitosterol

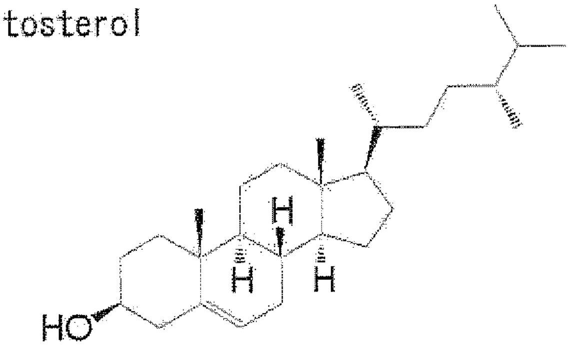

Campesterol

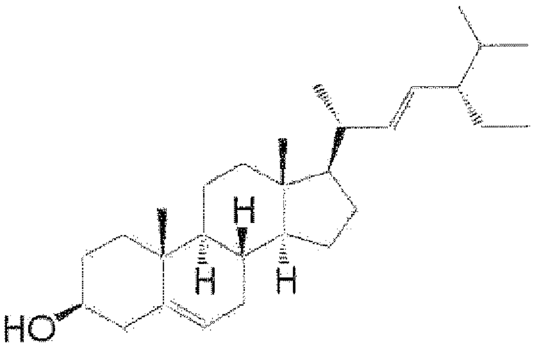

Stigmasterol

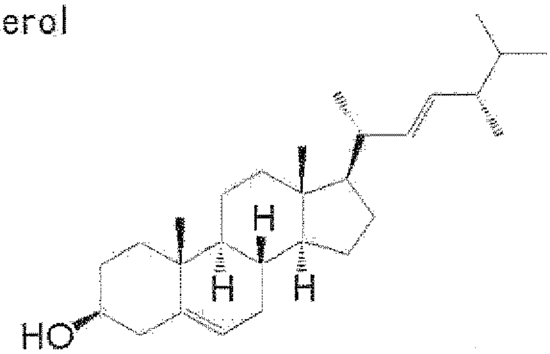

Brassicasterol

The stenone compound and the sterol compound can be obtained by synthesis or as a commercially available

product.
For example, phytostenone can be obtained as UNIFETH (manufactured by Toyo Hakko Co., Ltd.) and PEO-sterol can be obtained from NIKKOL BPS-20 (manufactured by Nikko Chemicals Co., Ltd.).
Each of the stenone compound and the sterol compound may be used singly, or plural kinds thereof may be used.
In the case of using one stenone compound singly, from the viewpoints of the dispersion stability of the natural-ceramide-containing particles, the content of the stenone compound is preferably 50% by mass or less with respect to the total mass of the oil-phase components contained in the ceramide dispersion, and more preferably 30% by mass or less.

(Oil Component as Active Ingredient)

In the case of using the ceramide dispersion according to the present invention for the use of a cosmetic product or a pharmaceutical product, it is preferable to include a functional material for a cosmetic product or a pharmaceutical product that is insoluble or poorly soluble in a water-soluble medium, particularly in water, as an oil component. It is possible to provide an excellent emollient effect, the anti-aging, the anti-oxidation effects for skin to the ceramide dispersion according to the present invention by including a functional oil component, for example, the below-mentioned astaxanthin in the ceramide dispersion according to the present invention.
The oil components usable in the present invention are not particularly limited as long as they are components that are soluble in an oil medium, and insoluble or poorly soluble in an aqueous medium, particularly, water. Preferable examples of the oil components include radical scavengers including an oil-soluble vitamin such as carotenoid and tocopherol, and a lipid, such as a coconut oil.
Meanwhile, being insoluble in an aqueous medium refers to a solubility of 0.01 g at 25°C with respect to 100mL of a water medium, and being poorly soluble in an aqueous medium refers to a solubility of more than 0.01 g to or less than 0.1g at 25°C with respect to 100mL of a water medium.

(Carotinoid)

As an oil component which is an active ingredient, any of carotinoids which may be a natural colorant may be preferably used.
The carotinoids which may be used in a composition for external application in the present invention are terpenoid colorants of any of yellow to red, and examples thereof include those of natural material derived from any of plants, algae, bacteria, and the like.
Further, the cartinoids which may be used in the present invention are not limited to natural materials, and any material obtained by a common method may be included in the scope of the cartinoids in the present invention. For examples, many carotenes described in the carotinoids as described below are produced by synthesis, and many commercially available β carotenes are also produced by synthesis.

Examples of carotenoids include hydrocarbons (carotenes), and oxidized alcohol derivatives thereof (xanthophylls).

Examples of carotenoids include actinioerythrol, astaxanthin, bixin, canthaxanthin, capsanthin, capsorubin, β-8'-apo-carotenal (apo-carotenal), β-12'-apo-carotenal, α-carotene, β-carotene, "carotene" (a mixture of α-carotene and β-carotene), γ-carotene, β-cryptoxanthin, lutein, lycopene, biolerythrine, zeaxanthin, and esters of those containing hydroxyl or carboxyl among the above.
Many of carotenoids exist in nature in the forms of a *cis*-isomer and a *trans*-isomer. Synthetic carotenoids are in the form of racemic mixtures in many cases.
In general, carotenoids can be extracted from plant materials. These carotenoids have various functions. For example, lutein extracted from the petal of marigold is widely used as a raw material for feed for domestic fowls, and has a function of coloring the skin and fat of domestic fowls, and eggs of domestic fowls.
The carotenoid used in the invention is particularly preferably at least one of astaxanthin or a derivative (such as ester) of astaxanthin (hereinafter collectively referred to as "an astaxanthin" or "astaxanthins"), which has, for example, an antioxidant effect, an anti-inflammatory effect, an anti-skin aging effect, and a whitening effect, and which is known as a yellow to red coloring agent.
Examples of natural products include red yeast Phaffia, green alga Haematococcus, oceanic bacteria, and Antarctic krill, and extracts such as extracts from cultures of such natural products.
The astaxanthins may be contained, in the ceramide dispersion of the invention, as an astaxanthin-containing oil obtained by separation/extraction (and purification as appropriate, if necessary) from the above-described other natural products containing astaxanthins.
As the astaxanthins, those extracted from Haematococcus alga (hereinafter sometimes referred to as Haematococcus alga extract) are particularly preferable in terms of quality and productivity.

The at least one of astaxanthin or an ester thereof (an astaxanthin) may be contained, in the ceramide dispersion of the invention, as an astaxanthin-containing oil isolated and extracted from a natural product containing at least one of astaxanthin or an ester thereof. Examples of the astaxanthin-containing oil include extracts from cultures obtained by culturing red yeast Phaffia, green alga Haematococcus, oceanic bacteria, or the like, and extracts from Antarctic krill or the like.
The Haematococcus alga extract (pigment derived from Haematococcus alga) is known to be different from pigments derived from krill or synthesized astaxanthins in terms of the types and contents of esters.
Astaxanthin that can be used in the invention may be any of the above extracts, or a product obtained by suitably purifying the extract as necessary, or a synthetic product.
As the astaxanthins, a product extracted from Haematococcus alga (hereinafter, sometimes referred to as a Haematococcus alga extract) is particularly preferable in terms of quality and productivity.
Specific origins of Haematococcus alga extracts that can be used in the invention include *Haematococcus pluvialis, Haematococcus lacustris, Haematococcus capensis, Haematococcus droebakensis,* and *Haematococcus zimbabwiensis.*
Widely-marketed Haematococcus alga extracts may be used in the invention. Examples thereof include ASTOTS-S, ASTOTS-2.5O, ASTOTS-5O, ASTOTS-10O, etc., which are manufactured by Takeda Shiki Co., Ltd.; ASTAREAL oil 50F, ASTAREAL oil 5F, etc., manufactured by Fuji Chemical Industry Co., Ltd.; and BIOASTIN SCE7 etc. manufactured by Toyo Koso Kagaku Co., Ltd.
The content, in terms of pure pigment content, of astaxanthins in the Haematococcus alga extract that can be used in the invention is preferably from 0.001% by mass to 50% by mass, and more preferably from 0.01% by mass to 25% by mass, from the viewpoint of the handling properties at the time of producing the ceramide dispersion.
The Haematococcus alga extract that can be used in the invention contains astaxanthin or esters thereof as a pure pigment, similarly to the pigment described in JP-A No. 2-49091. The proportion of esters is generally 50% by mol or higher, preferably 75% by mol or higher, and more preferably 90% by mol or higher.
More detailed explanations are described in "Astaxanthin-no-kagaku" (Chemistry of Astaxanthin), 2005, at the following internet site: <URL:http://www.astaxanthin.co.jp/chemical/basic.htm>

(Oils and fats)

Examples of oils and fats that are used as other oil components include oils, which are liquid at normal temperature (fatty oils), and fats, which are solid at normal temperature (fats).
Examples of liquid oils include olive oil, camellia oil, macadamia nuts oil, castor oil, avocado oil, evening primrose oil, turtle oil, corn oil, mink oil, rapeseed oil, yolk oil, sesame oil, persic oil, wheat germ oil, sasanqua oil, linseed oil, safflower oil, cotton seed oil, perilla oil, soybean oil, peanut oil, tea seed oil, kaya oil, rice bran oil, Chinese tung oil, Japanese tung oil, jojoba oil, germ oil, triglycerin, glycerin trioctanoate, glycerin triisopaltimate, salad oil, safflower oil (ref carthanus oil), palm oil, coconut oil, peanut oil, almond oil, hazelnut oil, walnut oil, and grape seed oil.
Examples of solid fats include beef tallow, hydrogenated beef tallow, neat's-foot tallow, beef bone tallow, mink oil, egg yolk oil, lard, horse fat, mutton tallow, hydrogenated oil, cacao fat, coconut oil, hydrogenated coconut oil, palm oil, hydrogenated palm oil, Japan wax, Japan wax kernel oil, and hydrogenated castor oil.
Among them, it is preferable to use coconut oil, which is a medium chain fatty acid triglyceride, from viewpoints of the dispersion particle diameter and the stability of the external composition.
In the invention, commercially available products may be used as the oils and fats. In the invention, the oils and fats may be used singly, or in mixture.
Examples of compounds having a phenolic OH include polyphenols (such as catechin), guaiac gum, nordihydroguaiaretic acid (NDGA), gallic esters, BHT (butylhydroxytoluene), BHA (butylhydroxyanisol), vitamine E compounds, and bisphenols. Examples of gallic esters include propoyl gallate, butyl gallate, and octyl gallate.
Examples of amine compounds include phenylene diamine, diphenyl-p-phenylene diamine, and 4-amino-p-diphenylamine. Diphenyl-p-phenylene diamine or 4-amino-p-diphenylamine is more preferable.
Examples of oil-soluble derivatives of ascorbic acid and erythorbic acid include L-ascorbyl stearate, L-ascorbyl tetraisopalmitate, L-ascorbyl palmitate, erythorbyl palmitate, and erythorbyl tetraisopalmitate.
Among the above, vitamine E compounds are particularly preferably used in consideration of high safety and excellent antioxidant function thereof.
Examples of vitamine E compounds are not particularly limited, and include a group of compounds consisting of tocopherol and derivatives thereof, and a group of compounds consisting of tocotrienol and derivatives thereof. These may be used singly, or in combination of two or more thereof. It is also permissible to use at least one compound selected from the group of compounds consisting of tocopherol and derivatives thereof, and at least one compound selected from the group of compounds consisting of tocotrienol and derivatives thereof in combination.
The group of compounds consisting of tocopherol and derivatives thereof include dl-α-tocopherol, dl-β-tocopherol, dl-γ-tocopherol, dl-δ-tocopherol, dl-α-tocopherol acetate, dl-α-tocopherol nicotinate, dl-α-tocopherol linoleate, and dl-α-tocopherol succinate. Among them, dl-α-tocopherol, dl-β-tocopherol, dl-γ-tocopherol, dl-δ-tocopherol, and mixtures thereof (mix trocophenols) are more preferable. Acetic esters thereof are preferably used as tocopherol derivatives. The group of compounds consisting of tocotrienol and derivatives thereof include α-tocotrienol, β-tocotrienol, γ-tocotrienol, and δ-tocotrienol. Acetic esters thereof are preferably used as tocotrienol derivatives. Tocotrienol is a tocopherol analogue compound contained in wheat, barley, rice bran, palm oil, and the like. Tocotrienol has three double bonds in the side chain portion of tocopherol, and has excellent antioxidant properties.

These vitamine E compounds are preferably contained, as oil-soluble antioxidants, particularly in the oil phase of the external composition, in which case the vitamine E compounds can effectively perform the function of preventing oxidation of oil components. Among the vitamin E compounds, it is preferable to include at least one selected from the group of compounds consisting of tocotrienol and derivatives thereof, from the viewpoint of antioxidant effects.

Considering the application to pharmaceutical products and cosmetics, the content of other oil components (such as those described above) in the ceramide dispersion of the invention when the other oil components are used is preferably from 0.1% by mass to 50% by mass, more preferably from 0.2% by mass to 25% by mass, and still more preferably from 0.5% by mass to 10% by mass, from the viewpoints of dispersion particle diameter and emulsion stability. When the content of oil components is 0.1% by mass or higher, the efficacy of active ingredients can be performed sufficiently, thereby facilitating applications of the ceramide dispersion to pharmaceutical products and cosmetics. When the content is 50% by mass or lower, an increase in the dispersion particle diameter and reduction of emulsion stability are suppressed, and a stable dispersion can be obtained.

(Surfactant)

The ceramide dispersion of the invention includes a surfactant. Specifically, the ceramide dispersion includes a polyglycerin fatty acid ester having a HLB of from 10 to 16 (hereinafter referred to as "specific polyglycerin fatty acid ester", as appropriate), which is a surfactant, as an essential component, from the viewpoint of emulsion stability. The ceramide dispersion may include the polyglycerine fatty acid ester in the oil phase.

As described above, the dispersion stability of natural-ceramide-containing particles, which are oil-phase dispersion particles, can be improved by including the specific stenone compound or the specific sterol compound in the oil phase as another oil component. However, in the invention, such another oil component is non-essential, and there is a case in which only natural-ceramide-containing particles are used as a component used in the oil phase. Therefore, it is essential to use the specific polyglycerin fatty acid ester as a surfactant, from the viewpoint of improving the dispersion stability of the natural-ceramide-containing particles.

Needless to say, the specific stenone compound or the specific sterol compound may be used even in a case in which a surfactant such as the specific polyglycerin fatty acid ester is employed.

In the invention, a surfactant such as the specific polyglycerin fatty acid ester can greatly decrease the surface tension of the oil phase/aqueous phase in the ceramide dispersion, and thus can decrease the particle diameter of the natural-ceramide-containing particles. Therefore, the use thereof is preferable.

Here, HLB refers to a balance between hydrophilicity and hydrophobicity generally used in the field of surfactants, and a commonly-employed calculation formula, for example Kawakami's equation, can be used. In the invention, Kawakami's equation described below is employed.

$$HLB = 7 + 11.7 \log (Mw/Mo)$$

In the equation, Mw represents the molecular weight of the hydrophilic groups and Mo represents the molecular weight of the hydrophobic groups.

The HLB numerical values described in catalogs and the like may also be employed.

Further, as is clear from the formula, a surfactant having an arbitrary HLB value can be obtained using the additive property of HLB.

The ceramide dispersion of the invention needs to include, as a surfactant, at least one polyglycerin fatty acid ester having a HLB of from 10 to 16 (specific polyglycerin fatty acid ester). It is preferable that at least one of the at least one specific polyglycerine fatty acid ester is an ester of a polyglycerine having an average polymerization degree of 10 and a fatty acid having from 8 to 18 carbon atoms such as caprylic acid, capric acid, lauric acid, myristic acid, palmitic acid, stearic acid, oleic acid, or linoleic acid.

Preferable examples of specific polyglycerin fatty acid esters that can be employed in the invention include hexaglycerin monooleate, hexaglycerin monopalmitate, hexaglycerin monomyristate, hexaglycerin monolaurate,

decaglycerin monooleate, decaglycerin monostearate, decaglycerin monopalmitate, decaglycerin monomyristate, and decaglycerin monolaurate. These esters have HLB values of from 10 to 16.
Among them, decaglycerin monooleate (HLB=12), decaglycerin monostearate (HLB=12), decaglycerin monopalmitate (HLB=13), decaglycerin monomyristate (HLB=14), and decaglycerin monolaurate (HLB=16) are more preferable.
In the invention, these specific polyglycerin fatty acid esters may be used singly, or in combination of two or more thereof.
The specific polyglycerin fatty acid ester is most preferably decaglycerin monooleate, and examples thereof include Decaglycerin monolinoleate (HLB=12), decaglycerin monooleate (HLB=12), decaglycerin monostearate (HLB=12), decaglycerin monomyristate (HLB=14), and decaglycerin monolaurate (HLB=15.5).
As to the surfactant in the invention, it is preferable that one of the specific polyglycerin fatty acid ester and at least one polyglycerin fatty acid ester having HLB of from 5 to 15 and having different molecular structure from that of the one specific polyglycerin fatty acid ester are contained in combination. The polyglycerin fatty acid ester having a HLB of from 5 to 15 may be a polyglycerin fatty acid ester that is within the scope of the specific polyglycerin fatty acid ester, or may be a polyglycerin fatty acid ester that is outside the scope of the specific polyglycerin fatty acid ester.
In the invention, it is preferable that decaglycerin oleate and a polyglycerin fatty acid ester which has a glycerin polymerization degree of less than 10 and of which the fatty acid has from 12 to 18 carbon atoms are contained as surfactants. The polyglycerin fatty acid ester which has a glycerin polymerization degree of less than 10 and of which the fatty acid has from 12 to 18 carbon atoms is more preferably a poly glycerin fatty acid ester which has a HLB of from 5.0 to 15 and which is at least one selected from a hexaglycerin fatty acid ester or a tetraglycerin fatty acid ester.
Examples of hexaglycerin fatty acid esters and tetraglycerin fatty acid esters that are preferably employed with decaglycerin oleate include tetraglycerin monostearate (HLB=6), tetraglycerin monooleate (HLB=6), hexaglycerin monolaurate (HLB=14.5), hexaglycerin monomyristate (HLB=11), hexaglycerin monostearate (HLB=9), and hexaglycerin monooleate (HLB=9).
In a case in which decaglycerin oleate, and hexaglycerin fatty acid ester and/or tetraglycerin fatty acid ester, are used together in the invention, the content ratio thereof can be set as appropriate depending on the applications of the ceramide dispersion; the ratio of (decaglycerin fatty acid ester) / (tetraglycerin fatty acid ester and/or hexaglycerin fatty acid ester) is preferably in the range of from 1/0 to 1/1, more preferably 1/0.5, and still more preferably 1/0.25.
Commercially available products may be applied as polyglycerin fatty acid esters such as the specific polyglycerin fatty acid ester.
Examples of commercially available polyglycerin fatty acid esters include NIKKOL DGMS, NIKKOL DGMO-CV, NIKKOL DGMO-90V, NIKKOL DGDO, NIKKOL DGMIS, NIKKOL DGTIS, NIKKOL TETRAGLYN 1-SV, NIKKOL TETRAGLYN 1-O, NIKKOL TETRAGLYN 3-S, NIKKOL TETRAGLYN 5-S, NIKKOL TETRAGLYN 5-O, NIKKOL HEXAGLYN 1-L, NIKKOL HEXAGLYN 1-M, NIKKOL HEXAGLYN 1-SV, NIKKOL HEXAGLYN 1-O, NIKKOL HEXAGLYN 3-S, NIKKOL HEXAGLYN 4-B, NIKKOL HEXAGLYN 5-S, NIKKOL HEXAGLYN 5-O, NIKKOL HEXAGLYN PR-15, NIKKOL DECAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV, NIKKOL DECAGLYN 1-LN, NIKKOL DECAGLYN 2-SV, NIKKOL DECAGLYN 2-ISV, NIKKOL DECAGLYN 3-SV, NIKKOL DECAGLYN 3-OV, NIKKOL DECAGLYN 5-SV, NIKKOL DECAGLYN 5-HS, NIKKOL DECAGLYN 5-IS, NIKKOL DECAGLYN 5-OV, NIKKOL DECAGLYN 5-O-R, NIKKOL DECAGLYN 7-S, NIKKOL DECAGLYN 7-O, NIKKOL DECAGLYN 10-SV, NIKKOL DECAGLYN 10-IS, NIKKOL DECAGLYN 10-OV, NIKKOL DECAGLYN 10-MAC, and NIKKOL DECAGLYN PR-20, which are manufactured by Nikko Chemicals Co., Ltd.;
RYOTO-POLYGLYESTER L-7D, L-10D, M-10D, P-8D, SWA-10D, SWA-15D, SWA-20D, S-24D, S-28D, O-15D, O-50D, B-70D, B-100D, ER-60D, LOP-120DP, DS13W, DS3, HS11, HS9, TS4, TS2, DL15, and DO13, which are manufactured by Mitsubishi Chemical Foods Co., Ltd.;
SUN SOFT Q-17UL, SUN SOFT Q-14S, and SUN SOFT A-141C, which are manufactured by Taiyo Kagaku CO., LTD.; and
POEM DO-100 and POEM J-0021, which are manufactured by Riken Vitamin Co., Ltd.
Among the above, NIKKOL DECAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV, NIKKOL DECAGLYN 1-LN, RYOTO-POLYGLYESTER L-7D, L-10D, M-10D, P-8D, SWA-10D, SWA-15D, SWA-20D, S-24D, S-28D, O-15D, O-50D, B-70D, B-100D, ER-60D, and LOP-120DP are preferable.
Further, in the invention, cationic, anionic, amphoteric, and nonionic surfactants other than the specific polyglycerin fatty acid ester described above, may be used. Such other surfactants are not particularly limited, and nonionic surfactants are preferable. Examples of nonionic surfactants include other glycerin fatty acid esters, organic acid monoglycerides, polyglycerin fatty acid esters, propyleneglycol fatty acid esters, polyglycerin condensed ricinoleic acid esters, sorbitan fatty acid esters, sucrose fatty acid esters, and polyoxyethylene sorbitan fatty acid esters. Sorbitan fatty acid esters, sucrose fatty acid esters, and polyoxyethylene sorbitan fatty acid esters are more preferable. Further, the surfactants need not be highly purified products obtained by, for example, distillation, and may be reaction mixtures.
The fatty acid in the sorbitan fatty acid ester has preferably 8 or more carbon atoms, and more preferably 12

or more carbon atoms. Preferable examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate.
In the invention, these sorbitan fatty acid esters may be used singly, or in mixture.

Examples of commercial products of sorbitan fatty acid esters include NIKKOL SL-10, SP-10V, SS-10V, SS-10MV SS-15V, SS-30V, SI-10RV, SI-15RV, SO-10V, SO-15MV, SO-15V, SO-30V, SO-10R, SO-15R, SO-30R, and SO-15EX, which are manufactured by Nikko Chemicals Co., Ltd., SORGEN 30V, 40V, 50V, 90, and 110, which are manufactured by DAI-ICHI KOGYO SEIYAKU CO.,LTD., and RHEODOL AS-10V, AO-10V, AO-15V, SP-L10, SP-P10, SP-S10V, SP-S30V, SP-O10V, and SP-O30V, which are manufactured by Kao Corporation.

The fatty acid in the sucrose fatty acid ester has preferably 12 or more carbon atoms, and more preferably from 12 to 20 carbon atoms.
Preferable examples of the sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. Among them, sucrose monooleate, sucrose monostearate, sucrose monopalmitate, sucrose monomyristate, and sucrose monolaurate are more preferable.
In the invention, these sucrose fatty acid esters may be used singly, or may in mixture.

Examples of commercial products of sucrose fatty acid esters include RYOTO SUGAR ESTER S-070, S-170, S-270, S-370, S-370F, S-570, S-770, S-970, S-1170, S-1170F, S-1570, S-1670, P-070, P-170, P-1570, P-1670, M-1695, O-170, O-1570, OWA-1570, L-195, L-595, L-1695, LWA-1570, B-370, B-370F, ER-190, ER-290, and POS-135, which are manufactured by Mitsubishi Chemical Foods Co., Ltd.; and DK ester SS, F160, F140, F110, F90, F70, F50, F-A50, F-20W, F-10, and F-A10E, and COSMELIKE B-30, S-10, S-50, S-70, S-110, S-160, S-190, SA-10, SA-50, P-10, P-160, M-160, L-10, L-50, L-160, L-150A, L-160A, R-10, R-20, O-10, and O-150, which are manufactured by DAI-ICHI KOGYO SEIYAKU CO.,LTD.
Among the above, RYOTO SUGAR ESTER S-1170, S-1170F, S-1570, S-1670, P-1570, P-1670, M-1695, O-1570, and L-1695, DK ester SS, F160, F140, and F110, and COSMELIKE S-110, S-160, S-190, P-160, M-160, L-160, L-150A, L-160A, and O-150, are preferable.

The fatty acid in the polyoxyethylene sorbitan fatty acid ester has preferably 8 or more carbon atoms, and more preferably 12 or more carbon atoms. The length of ethylene oxide (addition mole number) of the polyoxyethylene is preferably from 2 to 100, and more preferably from 4 to 50.
Preferable examples of polyoxyethylene sorbitan fatty acid esters include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate, and polyoxyethylene sorbitan trioleate.
These polyoxyethylene sorbitan fatty acid esters may be used singly, or in mixture.

Examples of commercial products of polyoxyethylene sorbitan fatty acid esters include NIKKOL TL-10, NIKKOL TP-10V, NIKKOL TS-10V, NIKKOL TS-1 0MV, NIKKOL TS-106V, NIKKOL TS-30V, NIKKOL TI-10V, NIKKOL TO-10V, NIKKOL TO-10MV, NIKKOL TO-106V, and NIKKOL TO-30V, which are manufactured by Nikko Chemicals Co., Ltd.; RHEODOL TW-L106, TW-L120, TW-P120, TW-S106V, TW-S120V TW-S320V, TW-O106V, TW-O120V, TW-O320V, and TW-IS399C, and RHEODOL SUPER SP-L10 and TW-L120, which are manufactured by Kao Corporation; and SORGEN TW-20, TW-60V, and TW-80V, which are manufactured by DAI-ICHI KOGYO SEIYAKU CO.,LTD.

In the invention, a lecithin may be used together with the surfactant. Lecithins that can be used in the invention contain a glycerin skeleton, a fatty acid residue, and a phosphoric acid residue as essential constituents, to which a base, a polyhydric alcohol, and the like are bonded; lecithins are also referred to as phospholipids. Since lecithins have a hydrophilic group and a hydrophobic group in a molecule thereof, lecithins have been widely used as emulsifying agents in the fields of foods, pharmaceuticals, and cosmetics from the past.

Industrially, those having a lecithin purity of 60% or higher are used as lecithins, and can be used in the invention. However, from the viewpoints of formation of oil droplets having very small particle diameter and stability of functional oil components, products that are generally referred to as high-purity lecithins are preferable, and the high-purity lecithins may have a lecithin purity of 80% or higher, and more preferably 90% or higher.

Examples of lecithins include various conventional products extracted and separated from living bodies of plants, animals and microorganisms.
Examples of such lecithins include various lecithins derived from, for example, plants such as soybean, corn, peanut, rapeseed, and wheat, animals such as egg yolk and cow, and microorganisms such as colon bacillus.
Examples of compound names of lecithins include phosphatidic acid, phosphatidylglycerin, phosphatidylinositol, phosphatidylethanolamine, phosphatidylmethylethanolamine, phosphatidylcholine, phosphatidylserine, bisphosphatidic acid, glycerolecithins such as diphosphatidylglycerin (cardiolipin), and sphingolecithins such as sphingomyelin.
In the invention, usable lecithins include not only the above high-purity lecithins, but also hydrogenated lecithins, enzymatically-decomposed lecithins, enzymatically-decomposed, hydrogenated lecithins, and hydroxylecithins. These lecithins usable in the invention may be used singly, or in the state of a mixture of two or more thereof.

In the ceramide dispersion of the invention, the content ratio of natural ceramide to surfactant may be adjusted as appropriate, in accordance with the type of surfactant. The mass of surfactant is preferably from 0.1 to 2 times the total mass of natural ceramide, more preferably from 0.5 to 1.75 times the total mass of natural ceramide, and most preferably from 0.75 to 1.5 times the total mass of natural ceramide.

A dispersion having a fine particle diameter can be obtained by setting the surfactant amount to be from 0.1 to 2 times the total mass of natural ceramide.

In terms of lower tendency toward heavy foaming, it is preferable that the surfactant amount is 2 times the total amount of natural ceramide or less. In a case in which the surfactant amount is less than 0.1 times the total amount of natural ceramide, the volume average particle diameter of dispersion particles may exceed 100 nm, which is not preferred due to opaqueness of the dispersion or composition.

(Water-soluble Organic Solvent)

The ceramide dispersion of the invention preferably contains a water-soluble organic solvent.

The water-soluble organic solvent in the present invention as an oil phase containing natural component is used for mixing with the aqueous solution described below. The aqueous organic solvent is also a main component of an extraction liquid with which the natural component is extracted. That is, in the invention, the natural ingredient is used and mixed with the aqueous solution in the state in which the natural ingredient has been extracted into the extraction liquid containing the water-soluble organic solvent as the main component.

The term "water-soluble organic solvent" as used in the invention refers to an organic solvent which has a solubility in water (at 25°C) of 10% by mass or higher. From the viewpoint of the stability of the resultant emulsion or dispersion, the solubility in water is preferably 30% by mass or higher, and more preferably 50% by mass or higher.

The water-soluble organic solvent may be used singly or as a mixed solvent of plural water-soluble organic solvents. Further, the water-soluble organic solvent may be used in the form of a mixture with water. When the water-soluble organic solvent is used as a mixture with water, the content of the water-soluble organic solvent in the mixture is preferably at least 50% by volume or higher, and more preferably 70% by volume or higher.

Although the water-soluble organic solvent is preferably used in order to mix oil-phase components and to prepare an oil phase, the water-soluble organic solvent is not included in the scope of the term "oil components" as used in the invention.

Examples of the water-soluble organic solvent include methanol, ethanol, 1-propanal, 2-propanol, 2-butanol, acetone, tetrahydrofuran, acetonitrile, methyl ethyl ketone, dipropylene glycol monomethyl ether, methyl acetate, methyl acetoacetate, N-methylpyrrolidone, dimethyl sulfoxide, ethyleneglycol, 1,3-butanediol, 1,4-butanediol, propyleneglycol, diethyleneglycol, triethyleneglycol, and the like, and mixtures thereof. Among these, ethanol, propyleneglycol, and acetone are preferable, and ethanol and a mixture of ethanol and water are more preferable, when the application thereof is limited to foods.

(Polyhydric alcohol)

It is preferable for the ceramide composition of the invention to contain a polyhydric alcohol from the viewpoints of particle diameter, stability, and preservability.

Polyhydric alcohols have a moisturizing function, a viscosity control function, etc. Polyhydric alcohols also have a function of decreasing the surface tension between water and oil and fat components, thereby facilitating the spreading of the interface and the formation of fine and stable particles.

As described above, inclusion of a polyhydric alcohol in the ceramide dispersion is preferable since it allows the dispersion particle diameter of the ceramide dispersion to be decreased to be very small, and allows the very small particle diameter to be maintained stably for a long time.

Moreover, the addition of a polyhydric alcohol decreases the water activity of the ceramide dispersion, thereby allowing microorganism propagation to be suppressed.

Dihydric or higher-hydric alcohols can be used as polyhydric alcohols usable in the invention, without particular limitations.

Examples of polyhydric alcohols include glycerin, diglycerin, triglycerin, polyglycerin, 3-methyl-1,3-butanediol, 1,3-butyleneglycol, isopreneglycol, polyethyleneglycol, 1,2-pentanediol, 1,2-hexanediol, propyleneglycol, dipropyleneglycol, polypropyleneglycol, ethyleneglycol, diethyleneglycol, pentaerythritol, neopentylglycol, maltitol, reduced starch syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitol, sorbitan, trehalose, amylolysis sugar, and amylolysis sugar reduced alcohol. These may be used singly, or in the form of a mixture of two or more thereof.

Further, the polyhydric alcohol is preferably a polyhydric alcohol having 3 or more hydroxyl groups in one

molecule thereof. Use of a polyhydric alcohol having 3 or more hydroxyl groups in one molecule thereof can effectively decrease the interfacial tension between an aqueous solvent and oil and fat components, thereby allowing the formation of finer and more stable particles. As a result, it is possible to further increase the intestinal absorption of the ceramide dispersion of the invention in a case of food applications, and the transdermal absorption of the ceramide dispersion in the case of transdermal pharmaceutical applications and cosmetic applications.

In particular, use of glycerin, among the polyhydric alcohols satisfying the conditions described above, is preferable since it further decreases the particle diameter of dispersion particles (natural-ceramide-containing particles) in the ceramide dispersion, and allows the small particle diameter to be maintained stably for a long time.

The content of polyhydric alcohol is preferably from 5 to 60 % by mass, more preferably from 10 to 55 % by mass, and further preferably from 30 to 50 % by mass, relative to the total mass of the ceramide dispersion, from the viewpoints of the viscosity of the ceramide dispersion as well as the particle diameter, stability, and perservability mentioned above.

A polyhydric alcohol content of 5 % by mass or higher is preferable in terms of the facilitating the provision of sufficient storage stability, irrespective of the type and the content of the oil and fat components. A polyhydric alcohol content of 60 % by mass or lower is preferable in terms of maximizing the effects and making it easy to prevent the viscosity of the ceramide dispersion from becoming high.

The ceramide dispersion of the invention may further include, as necessary, other additives that are usually employed in external compositions (compositions for external applications), such as various ingredients having medicinal poperties, preservatives, and colorants, as long as the effects of the invention are not impaired.

[Other Components]

According to the purpose, the ceramide dispersion of the invention may include, as appropriate, components that are employed in external compositions such as skin external preparations, in addition to the above components.

Examples of the additive compounds include moisturizing agents such as glycine betaine, xylitol, trehalose, urea, neutral amino acids, and basic amino acids, substances having medicinal properties such as Allantoin, organic powders such as cellulose powder, nylon powder, crosslinked slicone powder, crosslinkied methylpolysiloxane, porous cellulose powder, and porous nylon powder, inorganic powders such as anhydrous silica, zinc oxide, and titanium oxide, freshners such as menthol and camphor, plant extracts, pH bluffers, antioxidants, UV absorbers, preservatives, perfumes, microbicides, and colorants.

In the ceramide dispersion of the invention, in a case in which natural-ceramide-containing particles are used in the oil phase together with other oil components, the particle diameter of the dispersion particles contained as the oil phase can be controlled by factors such as the agitation conditions (shear force, temperature, pressure), usage conditions of the micromixer, and the ratio between the oil phase and the aqueous phase employed in the method of producing a ceramide dispersion described below, as well as the factors related to the components contained in the ceramide dispersion, as a result of which desired fine oil-phase particles of 100 nm or less can be obtained.

<Method of Producing Ceramide Dispersion>

The ceramide dispersion of the invention includes at least natural-ceramide-containing particles and at least one surfactant including the specific polyglycerin fatty acid ester. In general, the ceramide dispersion has a configuration in which an oil phase formed from natural-ceramide-containing particles or formed from at least one other oil component and natural-ceramide-containing particles is dispersed in an aqueous phase containing a surfactant.

A method of producing the dispersion includes:

individually preparing an aqueous phase, and an oil phase containing a natural ceramide and the like and at least one surfactant including the specific polyglycerin fatty acid ester or an oil phase containing a natural ceramide and the like, at least one surfactant including the specific polyglycerin fatty acid ester, and at least one other oil component, and

subjecting the prepared oil phase and the prepared aqueous phase to emulsification, thereby forming natural-ceramide-containing particles in the aqueous phase.

The viscosity of the aqueous phase is preferably 30 mPa·s or lower from the viewpoint of fining the natural-ceramide-containing particles.

From the viewpoint of fining the particles, the forming of the natural-ceramide-containing particles by emulsification preferably includes individually passing the oil phase and the aqueous phase, which have been separately prepared, through a microchannel of which the narrowest portion has a cross-sectional area of from 1 $\mu m^2$ to 1 $mm^2$, and then combining and mixing the oil phase and the aqueous phase. The preparation temperature can be changed in

accordance with the boiling point of the solvent to be used, and a preparation temperature of from 20°C to 80°C is usually preferable, and a preparation temperature of from 20°C to 60°C is more preferable. As described above, a method employing a high-pressure emulsification method in which the individually prepared oil phase and aqueous phase are mixed, and a high shear force of 100 MPa or higher is applied thereto, is also preferable.
A ceramide dispersion in which natural-ceramide-containing particles having a volume average particle diameter of from 0.5 nm to 100 nm are dispersed can be obtained thereby.

The method of producing the ceramide dispersion of the invention may include, for example, a) preparing an aqueous phase using an aqueous medium (e.g., water), b) preparing an oil phase containing a natural ceramide and the specific polyglycerin fatty acid ester, to which a water-soluble organic solvent, a specific stenone compound, and at least one other oil component (e.g., carotenoid) are optionally added, wherein the amount of natural ceramides is at least 50 % by mass relative to the total mass of the oil phase, and c) subjecting the oil phase and the aqueous phase to emulsification/dispersion by mixing the oil phase and the aqueous phase according to the method detailed below by using a micromixer, thereby providing a ceramide dispersion (emulsion) containing natural-ceramide-containing particles (dispersion particles) having a volume average particle diameter of from 0.5 nm to 100 nm.

In a case in which provision of a composition in the powder state by using the ceramide dispersion of the invention is desired, the composition in the powder state can be obtained by adding a step of drying the ceramide dispersion in the emulsion state obtained above by, for example, spray drying.
The components contained in the oil phase and the aqueous phase in the method of producing the ceramide dispersion are the same as the components of the ceramide dispersion of the invention described above, and preferable examples and preferable amounts thereof are also the same as in the case of the ceramide dispersion of the invention. The preferable combinations thereof described above are more preferred also in the method of producing the ceramide dispersion.

The ratio (by mass) of the oil phase to the water phase in the emulsification/dispersion is not particularly limited, and the oil phase/aqueous phase ratio (in terms of % by mass) is preferably from 0.1/99.9 to 50/50, more preferably from 0.5/99.5 to 30/70, and further preferably from 1/99 to 20/80.
A oil phase/aqueous phase ratio within the above range is preferable since it provides sufficient content of active ingredients and sufficient emulsion stability for practical use.

[Micromixer]

In order to stably form natural-ceramide-containing particles of from 0.5 nm to 100 nm, the production method employed for the production of the ceramide dispersion of the invention is preferably a production method of individually passing the oil phase and the aqueous phase through a microchannel of which the narrowest portion has a cross-sectional area of from 1 $\mu m^2$ to 1 $mm^2$, and thereafter combining and mixing the aqueous phase and the oil phase.
The mixing of the oil phase and the aqueous phase is preferably mixing by collision of counter flows, from the viewpoint of obtaining finer dispersion particles.
A most suitable device for mixing by collision of counter flows is a counter-collision micromixer. A micromixer is generally a device which mixes two different liquids in a microspace; one of the liquids is an organic solvent phase containing a functional oil component, and the other liquid is an aqueous phase which is an aqueous solution.
The application of a micromixer to preparation of an emulsion having a small particle diameter, which is a microchemical process, exhibits relatively low energy consumption, generates less heat, and enables provision of a favorable emulsion or dispersion that has substantially uniform particle diameters and has excellent storage stability compared to usual agitation emulsification dispersing methods or high-pressure homogenizer emulsification dispersing methods. The application of a micromixer is a most suitable method for emulsification when a natural ingredient that is susceptible to thermal deterioration is contained.

The gist of an emulsification or dispersing method using a micromixer lies in individually distributing the aqueous phase and the oil phase to microspaces, and contacting or colliding the aqueous and oil phases distributed in the respective microspaces. This method is clearly different from a membrane emulsification method or a microchannel emulsification method, in which only one phase is distributed to microspaces and the other phase is supplied in bulk. Indeed, when only one phase is distributed to microspaces, the effects of the invention cannot be obtained. Known micromixers have various structures. In terms of flow and mixing in microchannels, two kinds of method can be exemplified: one method is a method of mixing while maintaining a laminar flow, and the other is a method of mixing while disturbing the flow, that is, in the state of a turbulent flow. In the method of mixing while maintaining a laminar flow, the efficiency of mixing is improved by adjusting the depth dimension of flow channels to be larger than the width dimension of the flow channels so as to increase the boundary area between the two liquids as far as possible, and to reduce the thickness of both layers. A method employing a multilayer flow formed by alternately flowing the two liquids from multiply-divided inlets has also been proposed.

A method of distributing the individual liquids into narrow flow channels and flowing the liquids at a relatively

high speed is generally employed as a method of mixing in the turbulent flow state. A method in which one liquid is sprayed into the other liquid that has been introduced into microspaces using an arrayed micro-nozzle has also been proposed. Furthermore, a particularly excellent mixing effect can be obtained by a method in which liquids flowing at high speeds are forcibly contacted with each other using various means. The former method using a laminar flow generally produces particles having large diameters and a relatively uniform particle diameter distribution. In the latter method using a turbulent flow, there is a possibility that a very fine emulsion is obtained, and thus the method using a turbulent flow is preferable in terms of stability and transparency in many cases. Representative methods using a turbulent flow include a method using a slit-interdigital-type micromixer and a method using a collision-type micromixer. A slit-interdigital-type micromixer, as typified by a mixer manufactured by IMM Gmbh, has a structure in which two interdigital flow channels are faced and arranged so as to alternately protrude towards the other flow channel.

A collision-type micromixer, represented by a KM mixer, has a structure in which forcible contact is conducted by utilizing kinetic energy. Specifically, collision-type micromixers include a central collision-type micromixer disclosed by Nagasawa et al. ("H. Nagasawa et al., Chem. Eng. Technol., 28, No.3, 324-330 (2005)"; JP-ANo. 2005-288254). An extremely fine emulsion or dispersion can be easily formed according to the method of counter-colliding the aqueous phase and the organic solvent phase due to extremely short mixing time thereof and instant formation of oil-phase droplets.

In the invention, in a case in which emulsification is performed by micro-mixing using a collision-type micromixer, the temperature during emulsification (emulsification temperature) is preferably such that the temperature of the other microspaces of the micromixer (the temperature at the micro-mixing part of the micromixer) during micro-mixing is preferably 80°C or lower, more preferably from 0°C to 80°C, and particularly preferably from 5°C to 75°C, from the viewpoint of uniformity of the particle diameter of the resultant emulsion. An emulsification temperature of 0°C or higher allows emulsification temperature control since the main component of the dispersion medium is water, and thus an emulsification temperature of 0°C or higher is preferable. The temperature of the microspaces of the micromixer is preferably maintained at 100°C or lower. When the temperature is maintained at 100°C or lower, the maintained temperature can be easily controlled, and micro-bumping phenomenon adversely affecting the emulsification performance can be prevented. The temperature is more preferably controlled to be maintained at a temperature of 80°C or lower.

The temperature at which the oil phase that is distributed to the microspaces of the micromixer is maintained, the temperature at which the aqueous phase that is distributed to the microspaces of the micromixer is maintained, and the temperature at which the microspaces of the micromixer are maintained are each independently preferably from 0°C to 50°C, and particularly preferably from 5°C to 25°C. Although the temperature at which the microspaces of the micromixer are maintained, the temperature at which the oil phase and the aqueous phase distributed to the microspaces of the micromixer are maintained, and the temperature at which the oil phase and the aqueous before distributed to the microspaces of the micromixer are maintained (i.e., the temperature at which the oil phase supply tank and the aqueous phase supply tank are maintained) may be different from one another, they are preferably the same temperature in the point of stability of mixing.

In the invention, it is particularly preferable that the temperatures at which the aqueous phase and the oil phase before and after distributed to the microspaces, the temperature at which the microspaces of the micromixer are maintained, and the temperature at which the other microspaces of the micromixer are maintained, are adjusted to a temperature higher than ambient temperature, and, after micro-mixing and emulsification, the oil-in-water emulsion obtained using the micromixer is cooled to ambient temperature after collection.

The cross-sectional area of the narrowest portion of the microspaces (flow channels) of the micromixer in the invention is from 1 $\mu m^2$ to 1 $mm^2$ and preferably from 500 $\mu m^2$ to 50,000 $\mu m^2$, from the viewpoints of decreasing the emulsion particle diameter and narrowing the particle diameter distribution.

The cross-sectional area of the narrowest portion of the microspaces (flow channels) for the aqueous phase in the micromixer in the invention is particularly preferably from 1,000 $\mu m^2$ to 50,000 $\mu m^2$ from the viewpoint of the stability of mixing.

The cross-sectional area of the narrowest portion of the microspaces (flow channels) for the oil phase in the micromixer is particularly preferably from 500 $\mu m^2$ to 20,000 $\mu m^2$ from the viewpoints of decreasing the emulsion particle diameter and narrowing the particle diameter distribution.

In the case of emulsifying/dispersing using a micromixer, the flow rates of the oil phase and the aqueous phase during emulsification/dispersing varies depending on the micromixer used; the flow rate of the aqueous phase is preferably from 10 ml/min to 500 ml/min, more preferably from 20 ml/min to 350 ml/min, and particularly preferably from 50 ml/min to 200 ml/min, from the viewpoints of decreasing the emulsion particle diameter and narrowing the particle diameter distribution.

The flow rate of the oil phase is preferably from 1 ml/min to 100 ml/min, more preferably from 3 ml/min to 50 ml/min, and particularly preferably from 5 ml/min to 50 ml/min, from the viewpoints of decreasing the emulsion particle diameter and narrowing the particle diameter distribution.

The values obtained by dividing the flow rates of both phases by the cross-sectional area of the microchannel, that is, the ratio (Vo/Vw) between the flow speeds of both phases is preferably in the range from 0.05 to 5 from the

viewpoints of forming finer emulsion particles and the design of the micromixer. Here, Vo represents the flow speed of the organic solvent phase containing a water-insoluble natural component, and Vw represents the flow speed of the aqueous phase. Furthermore, the flow speed ratio (Vo/Vw) is most preferably in the range of from 0.1 to 3 from the viewpoint of further fining the particles.

In addition, the liquid feed pressures of the oil phase and the aqueous phase are preferably from 0.030 MPa to 5 MPa and from 0.010 MPa to 1 MPa, respectively, and more preferably from 0.1 MPa to 2 MPa and from 0.02 MPa to 0.5 MPa, respectively, and particularly preferably from 0.2 MPa to 1 MPa and 0.04 MPa to 0.2 MPa, respectively. A liquid feed pressure of the aqueous phase of from 0.030 MPa to 5 MPa provides higher tendency toward maintenance of stable liquid feed rate. A liquid feed pressure of the oil phase of from 0.010 MPa to 1 MPa provides a higher tendency towards uniform mixing properties. Thus, the liquid feed pressure ranges are preferable.

In the invention, it is more preferable to combine the respective preferable examples of the flow rate, the liquid feed pressure, and the maintained temperature.

Next, a pathway from introduction of the aqueous phase and the oil phase into the micromixer until discharge thereof as an oil-in-water type emulsion is explained using an exemplary microdevice (Fig. 1) as one example of the micromixer in the invention.

As shown in Fig. 1, microdevice 100 consists of supply element 102, confluence element 104, and discharge element 106, each having a cylindrical form.

On a surface of supply clement 102 facing confluence element 104, annular channels 108 and 110, each having a rectangular cross-sectional shape, are arranged concentrically as channels for the oil phase and the aqueous phase of the invention. In supply element 102, bores 112 and 114 are provided which penetrate supply element 102 in the thickness (or height) direction thereof, and which leads to the respective annular channels.

In confluence element 104, bores 116 are provided which penetrates confluence element 104 in the thickness direction thereof. These bores 116 are arranged such that, when the elements are fastened to one another to form microdevice 100, ends 120 of bores 116 located on a surface of confluence element 104 facing supply element 102 open into annular channel 108. In the embodiment shown in the drawing, four bores 116 are provided, which are arranged at constant spacing along the circumferential direction of annular channel 108.

In confluence element 104, penetrating bores 118 are provided, similarly to bores 116. Bores 118 are provided so as to open into annular channel 110, similarly to bores 116. Bores 118 are arranged at constant spacing along the circumferential direction of annular channel 110, and bores 116 and bores 118 are alternately-arranged.

On surface 122 of confluent element 104 facing discharge element 106, microchannels 124 and 126 are provided. One end of each microchannel 124 or 126 is an opening portion of bore 116 or 118, and the other end is center 128 of surface 122. All microchannels extend from the bores towards this center 128, and join together at the center. The cross-sectional shape of the microchannels may be, for example, a rectangular shape.

In discharge element 106, bore 130 is provided which penetrates discharge element 106 in the thickness direction thereof so as to pass the center thereof. Therefore, this bore opens into center 128 of confluence element 104 at one end, and opens to the outside of the microdevice at the other end.

In the present microdevice 100, fluids A and B supplied from the outside of microdevice 100 to the ends of bores 112 and 114 flow into annular channels 108 and 110 via bores 112 and 114, respectively.

Annular channel 108 and bores 116 communicate with each other, and fluid A that has flowed into annular channel 108 enters microchannels 124 via bores 116. Annular channel 110 and bores 118 communicate with each other, and fluid B that has flowed into annular channel 110 enters microchannel s 126 via bores 118. After fluids A and B flow into microchannels 124 and 126, respectively, fluids A and B flow towards center 128, and join together.

The fluids that have joined together are discharged as a stream C to the outside of microdevice via bore 130.

Microdevice 100 may have the specifications described below.

Cross-sectional shape of annular channel 108: rectangular
width/depth/diameter: 1.5 mm/1.5 mm/25 mm
Cross-sectional shape of annular channel 110: rectangular
width/depth/diameter: 1.5 mm/1.5 mm/20 mm
Bore 112: diameter/length: 1.5 mm/10 mm (circular cross-section)
Bore 114: diameter/length: 1.5 mm/10 mm (circular cross-section)
Bore 116: diameter/length: 0.5 mm/4 mm (circular cross-section)
Bore 118: diameter/length: 0.5 mm/4 mm (circular cross-section)
Cross-sectional shape of microchannel 124: rectangular
width/depth/length/cross-sectional area: 350 $\mu$m/100 $\mu$m/12.5 mm/35,000 $\mu m^2$
Cross-sectional shape of microchannel 126: rectangular
width/depth/length/cross-sectional area: 50 $\mu$m/100 $\mu$m/10 mm/5,000 $\mu m^2$
Bore 130: diameter/length: 500 $\mu$m/10 mm (circular cross-section)

Preferable ranges of the sizes of the microchannels (124 and 126 in Fig. 1) at which the aqueous phase and the oil phase collide with each other are defined in consideration of the relationship with the flow rates of the aqueous phase and the oil phase.

The micromixers described in JP-ANo. 2004-33901 are also preferable for use in the invention.
Fig. 2 is a schematic cross-sectional view of a T-shaped microreactor, which illustrates an example of the mixing mechanism employed by a T-shaped microreactor. Fig. 3 is a conceptual diagram of a T-shaped microreactor, which illustrates an example of the mixing mechanism employed by a T-shaped microreactor.
Fig. 2 shows a cross-section of T-shaped flow channel 200 of the T-shaped microreactor. In T-shaped flow channel 200, a fluid flowing in from inlet 202a in the direction indicated by arrow D and a fluid flowing in from inlet 202b in the direction indicated by arrow E collide with each other at the central portion of T-shaped flow channel 200, as a result of which the fluids are mixed and form fine fluid particles. The fine fluid particles flow out from outlet 204 in the direction indicated by arrow F. The T-shaped microreactor is useful for mixing when the volume of the flow channel is small.

Fig. 3 shows the fluid mixing mechanism (concept) of another T-shaped microreactor 300. In the fluid mixing mechanism shown in Fig. 3, fluids flowing out of two flow channels 302a and 302b collide and mix with each other, thereby forming fine fluid particles. Specifically, a fluid at one side flows into flow channel 302a in the direction indicated by arrow G, and flows out in the direction indicated by arrow H. A fluid at another side flows into flow channel 302b in the direction indicated by arrow I, and flows out in the direction indicated by arrow J. The fluids that have flown out of flow channels 302a and 302b, respectively, collide and mix with each other, and are scattered in directions substantially orthogonal to the directions indicated by arrows G to J. The fluid mixing mechanism illustrated in flow channel diagram 3 causes the fluids dispersed by a technique such as atomization to collide and mix with each other. The collision and mixing makes the fluid finer, thereby providing a larger contact face.

In the production method applicable to the ceramide dispersion of the invention, the water-soluble organic solvent used is preferably removed after the emulsifying or dispersing through the microchannels. Examples of methods of removing the solvent include an evaporation method using a rotary evaporator, a flash evaporator, an ultrasound atomizer, or the like, and a membrane separation method using an ultrafiltration membrane, a reverse osmosis membrane, or the like; an ultrafiltration membrane method is particularly preferable.

An ultra filter (UF) is an apparatus that applies a pressure to a stock solution (a mixed aqueous solution of water, a high-molecular substance, a low-molecular substance, a colloidal substance, and the like) so as to apply the stock solution to the UF device, thereby separating the stock solution into two solutions - a filtrate (the low-molecular substance) and a concentrate (the high-molecular substance, the colloidal substance) - which can then be taken out.

An ultrafiltration membrane is a typical asymmetric membrane produced by the Loeb-Sourirajan method. Examples of polymer materials that can be used for ultrafiltration membranes include polyacrylonitrile, polyvinyl chloride-polyacrylonitrile copolymer, polysulfone, polyether sulfone, vinylidene fluoride, aromatic polyamide, and cellulose acetate. In recent years, ceramic membranes have also been employed. Unlike the reverse osmosis method and the like, the ultrafiltration method does not involve pre-treatment, and, therefore, fouling whereby polymers or the like deposit on the membrane face occurs. Accordingly, the membrane is usually washed with a chemical or hot water regularly. Thus, the membrane material should have resistance against chemicals and heat resistance. There are various types of membrane module for ultrafiltration membranes, such as a flat membrane type, a tubular type, a hollow fiber type, and a spiral type. The performance indicator of ultrafiltration membranes is a molecular weight cut off, and various membranes having molecular weight cut off values of from 1,000 to 300,000 are commercially available. Examples of commercially available membrane modules include, but are not limited to, MICROSA UF (Asahi Kasei Chemicals Corporation), and capillary-type element NTU-3306 (Nitto Denko Corporation).

In terms of removing solvent from the resultant emulsion, the membrane material is particularly preferably polysulfone, polyether sulfone, or aromatic polyamide, from the viewpoint of resistance against solvent. In regard to the membrane module form flat membranes are mainly employed on the laboratory scale, while membranes of a hollow fiber type and a spinal type are employed industrially. Hollow fiber type membranes are particularly preferable. Although the molecular weight cut off varies depending on the kind of active ingredient, membranes having molecular weight cut off values in the range of from 5,000 to 100,000 are commonly used.
Although the operation temperature can be from 0°C to 80°C, a temperature range of 10°C to 40°C is particularly preferable in view of degradation of active ingredients.

Examples of the laboratory-scale ultrafiltration apparatuses include ADVANTEC-UHP (ADVANTEC) and a flow-type labo-test unit RUM-2 (manufactured by Nitto Denko Corporation), in which flat membrane modules are used. Industrially, a plant can be constructed by arbitrarily combining the sizes and numbers of individual membrane modules so as to accord the required capacity. As a bench-scale unit, RUW-5A (manufactured by Nitto Denko Corporation) and the like are commercially available.

The production method applicable to the ceramide dispersion of the invention may further include a step of concentrating the obtained emulsion subsequent to the removal of the solvent. In regard to the concentration method, the same methods and devices as in the case of the solvent removal may be used, such as an evaporation method and

a filtration method.
A ultra-filtration method is preferable also in the case of the concentration. Although it is preferable that the same membrane as that used in the solvent removal can be used for the concentration, a ultrafiltration membrane having a different molecular weight cut off may be used, if necessary. It is also possible to increase the concentration efficiency by conducting the concentration at a temperature different from that employed in the solvent removal.
The ceramide dispersion (emulsion) obtained by the above-described mixing by a micromixer is an oil-in-water type emulsion. The volume average particle diameter (median diameter) of the dispersion particles in the emulsion is set to be from 1 nm to 100 nm in the method of producing an external composition according to the invention. The volume average particle diameter of the dispersion particles is more preferably from 1. nm to 50 nm from the viewpoint of the transparency of the resultant emulsion.
The particle diameters of the natural-ceramide-containing particles (dispersion particles) obtained by the production method described above can be measured using, for example, a commercial particle size distribution analyzer, and details thereof are as described above.

<Applications>

Since the ceramide dispersion of the invention can be prepared as a fine emulsion having excellent emollient effects due to the natural ceramide, the ceramide dispersion of the invention is used in various applications that accord with the functions of the natural ceramide.
Such applications widely include applications as pharmaceutical products (products for external application, dermatorogical preperations), cosmetics, and foods. Examples of pharmaceutical products include parenteral products such as suppository and application (externally applied dermatological preparations), and examples of cosmetics include skin care cosmetics (such as skin lotions, serums, milky lotions, and creams), sunscreen cosmetics, and make-up cosmetics such as lipsticks and foundation. However, the examples are not limited thereto.
In a case in which the ceramide dispersion of the invention is used in dermatologic preparations for external application and cosmetics, ingredients that can be added to pharmaceutical products and cosmetics may be added as appropriate.
When the ceramide dispersion of the invention is used in aqueous products such as skin lotions, serums, milky lotions, cream packs and masks, packs, shampoo cosmetics, fragrance cosmetics, liquid body cleaning preparations, UV care cosmetics, deodorants, oral health cosmetics, gels containing a painkiller or anti-inflammatory agent, and active ingredient-containing layers of patches containing an anti-inflammatory agent, products having transparent feeling can be obtained. Further, unfavorable phenomena such as precipitation, sedimentation, and neckling of insoluble substances under severe conditions such as long-term storage or sterilization treatment can be suppressed.
Disclosures of Japanese Patent Application No. 2008-141181 is incorporated herein by reference in its entirety. All publications, patent applications, and technical standards mentioned in this specification are herein incorporated by reference to the same extent as if each individual publication, patent application, or technical standard was specifically and individually indicated to be incorporated by reference.
In the present specification, numerical ranges defined by using an expression "from...to..." represents ranges inclusive of the numbers that respectively appear at the left and right of "to" as the minimum value and the maximum value, respectively.

EXAMPLES

Hereinbelow, the present invention are described in more detail with examples, but the present invention is not limited to the following examples as long as it does not depart from the gist of the invention. Meanwhile, unless otherwise described, "parts" refers to parts by mass.

(Example 1)

Each of the components described in the oil phase liquid 1 composition below is stirred at room temperature for one hour to prepare the oil phase liquid 1.

<Composition of Oil Phase Liquid 1>

| | |
|---|---|
| Ceramide 3 [natural ceramide, specific example 1-5] | 0.9 parts |
| Ceramide 6 [natural ceramide, specific example 1-7] | 1.1 parts |
| Decaglycerin monooleate (HLB = 12) | 2.0 parts |
| Ethanol [water-soluble organic solvent] | 76.0 parts |

The obtained oil phase liquid 1 (oil phase) and water (aqueous phase) are micro-mixed in a ratio (mass ratio) of 1:7 using a KM-type micro mixer 100/100, which is collision-type, to obtain Ceramide dispersion A. Meanwhile, the conditions used for the micro mixer are as follows:

Micro Channel-

Oil phase-side micro channel

Cross-sectional shape/ Width/ Depth/ Length = rectangle/ 70 μm/ 100 μm/ 10 mm
Water phase-side micro channel
Cross-sectional shape/ Width/ Depth/ Length = rectangle/ 490 μm/ 100 μm/10 mm

-Flow Rate-

The aqueous phase is injected along the outer circumference in a flux of 21.0 mL/min., and the oil phase is injected along the inner circumference in a flux of 3.0 mL/min. thereby performing micro mixing.

The obtained ceramide dispersion 1 is concentrated and adjusted to have the ceramide concentration of 1.0% by mass by desolvating to the extent that the ethanol concentration becomes 0.1% by mass or less using "EVAPOR (CEP-lab, manufactured by Okawara Corporation)", thereby obtaining the ceramide dispersion 1. Here, the ceramide concentration refers to a concentration based on the total mass of the solid content added to the oil phase.

(Example 2)

Ceramide dispersion 2 was obtained in the same manner as in example 1 except that the oil phase liquid 1 was replaced with the oil phase liquid 2 below.

<Composition of Oil Phase Liquid 2>

| | |
|---|---|
| Ceramide 3 [natural ceramide, specific example 1-5] | 0.9 parts |
| Ceramide 6 [natural ceramide, specific example 1-7] | 1.1 parts |
| Decaglycerin monooleate (HLB = 12) | 2.0 parts |
| Hexaglycerin monostearate (HLB =9) | 0.25 parts |
| Ethanol [water-soluble organic solvent] | 76.0 parts |

(Example 3)

Ceramide dispersion 3 was obtained in the same manner as in Example I except that the oil phase liquid 1 was replaced with the oil phase liquid 3 below.

<Composition of Oil Phase Liquid 3>

| | |
|---|---|
| Ceramide 3 [natural ceramide, specific example 1-5] | 0.9 parts |
| Ceramide 6 [natural ceramide, specific example 1-7] | 1.1 parts |
| Decaglycerin monooleate (HLB = 12) | 1.5 parts |
| Tetraglycerin monooleate (HLB = 6) | 0.5 parts |
| Ethanol [water-soluble organic solvent] | 76.0 parts |

(Example 4)

Ceramide dispersion 4 was obtained in the same manner as in Example 1 except that the oil phase liquid 1 was replaced with the oil phase liquid 4 below.

<Composition of Oil Phase Liquid 4>

| | |
|---|---|
| Ceramide 3 [natural ceramide, specific example 1-5] | 0.9 parts |
| Ceramide 6 [natural cerainide, specific example 1-7] | 1.1 parts |

(continued)

| | |
|---|---|
| <Composition of Oil Phase Liquid 4> | |
| Decaglycerin monooleate (HLB = 12) | 1.75 parts |
| Hexaglycerin monolaurate (HLB = 14.5) | 0.25 parts |
| Ethanol [water-soluble organic solvent] | 76.0 parts |

(Example 5)

Ceramide dispersion 5 was obtained in the same manner as in Example 1 except that the oil phase liquid 1 was replaced with the oil phase liquid 5 below.

| | |
|---|---|
| <Composition of Oil Phase Liquid 5> | |
| Ceramide 3 [natural ceramide, specific example 1-5] | 0.9 parts |
| Ceramide 6 [natural ceramide, specific example 1-7] | 1.1 parts |
| Decaglycerin monostearate (HLB =15) | 1.25 parts |
| Tetraglycerin monooleate (HLB = 6) | 0.75 parts |
| Ethanol [water-soluble organic solvent] | 76.0 parts |

(Example 6)

Ceramide dispersion 6 was obtained in the same manner as in Example 1 except that the oil phase liquid 1 was replaced with the oil phase liquid 6 below.

| | |
|---|---|
| <Composition of Oil Phase Liquid 6> | |
| Ceramide 3 [natural ceramide, specific example 1-5] | 0.9 parts |
| Ceramide 6 [natural ceramide, specific example 1-7] | 1.1 parts |
| Decaglycerin monostearate (HLB = 15) | 1.25 parts |
| Tetraglycerin monooleate (HLB = 6) | 1.25 parts |
| Cholesterol(trade name: SANSTEROL, manufactured by San-Ei Gen F.F.I., Inc.) | 2.0 parts |
| Ethanol [water-soluble organic solvent] | 76.0 parts |

(Example 7)

Ceramide dispersion 7 was obtained in the same manner as in Example I except that the oil phase liquid 1 was replaced with the oil phase liquid 7 below.

| | |
|---|---|
| <Composition of Oil Phase Liquid 7> | |
| Ceramide 3 [natural ceramide, specific example 1-5] | 0.9 parts |
| Ceramide 6 [natural ceramide, specific example 1-7] | 1.1 parts |
| Decaglycerin monooleate (HLB =12) | 1.25 parts |
| Hexaglycerin monostearate (HLB = 9) | 1.25 parts |
| Phospholipid (trade name: COATSOME NC21, manufactured by NOF Corporation) | 0.5 part |
| Ethanol [water-soluble organic solvent] | 76.0 parts |

(Example 8)

Ceramide dispersion 8 was obtained in the same manner as in Example 2 except that the oil phase liquid 2 was replaced with the oil phase liquid 8 below.

| | |
|---|---|
| <Composition of Oil Phase Liquid 8> | |
| Ceramide 1 [natural ceramide, specific example 1-2] | 0.5 parts |
| Ceramide 3 [natural ceramide, specific example 1-5] | 1.25 parts |
| Ceramide 6 [natural ceramide, specific example 1-7] | 1.25 parts |

(continued)

| | |
|---|---|
| <Composition of Oil Phase Liquid 8> | |
| Decaglycerin monooleate (HLB = 12) | 2.0 parts |
| Hexaglycerin monostearate (HLB= 9) | 0.25 parts |
| Ethanol [water-soluble organic solvent] | 76.0 parts |

(Comparative Example 1)

Ceramide dispersion 9 was obtained in the same manner as in Example I except that the oil phase liquid 1 was replaced with the oil phase liquid 9 below.

| | |
|---|---|
| <Composition of Oil Phase Liquid 9> | |
| Ceramide 3 [natural ceramide, specific example 1-5] | 0.9 parts |
| Ceramide 6 [natural ceramide, specific example 1-7] | 1.1 parts |
| Decaglycerin tristearic acid ester (HLB = 7.5) | 2.0 parts |
| Ethanol [water-soluble organic solvent] | 76.0 parts |

(Comparative Example 2)

Ceramide dispersion 10 was obtained in the same manner as in Example 1 except that the oil phase liquid 1 was replaced with the oil phase liquid 10 below.

| | |
|---|---|
| <Composition of Oil Phase Liquid 10> | |
| Ceramide 3 [natural ceramide, specific example 1-5] | 0.9 parts |
| Ceramide 6 [natural ceramide, specific example 1-7] | 1.1 parts |
| Hexaglycerin monolaurate (HLB = 14.5) | 1.5 parts |
| Ethanol [water-soluble organic solvent] | 76.0 parts |

(Comparative Example 3)

Ceramide dispersion 11 was obtained in the same manner as in Example 1 except that the oil phase liquid 1 was replaced with the oil phase liquid 11 below.

| | |
|---|---|
| <Composition of Oil Phase Liquid 11> | |
| Ceramide 3 [natural ceramide, specific example 1-5] | 0.9 parts |
| Ceramide 6 [natural ceramide, specific example 1-7] | 1.1 parts |
| Decaglycerin monostearate (HLB =15) | 1.25 parts |
| Hexaglycerin tristearic acid ester (HLB = 2.5) | 1.25 parts |
| Stearic acid | 3.0 parts |
| Ethanol [water-soluble organic solvent] | 76.0 parts |

(Comparative Example 4)

Ceramide dispersion 12 was obtained in the same manner as in Example 1 except that the oil phase liquid 1 was replaced with the oil phase liquid 12 below.

| | |
|---|---|
| <Composition of Oil Phase Liquid 12> | |
| Ceramide 2 [natural ceramide, specific example 1-5] | 1.5 parts |
| Ceramide glycolipid (rice-derived) | 0.5 parts |
| Phytosphingosine | 0.5 parts |
| Ethanol [water-soluble organic solvent] | 76.0 parts |

(Comparative Example 5)

Ceramide dispersion 13 was obtained in the same manner as in Example 1 except that the oil phase liquid 1 was replaced with the oil phase liquid 13 below.

| <Composition of Oil Phase Liquid 13> | |
|---|---|
| Ceramide 3 [natural ceramide, specific example 1-5] | 0.9 parts |
| Ceramide 6 [natural ceramide, specific example 1-7] | 1.1 parts |
| Sorbitan monostearate POE (HLB = 14.9) | 2.0 parts |
| Ethanol [water-soluble organic solvent] | 76.0 parts |

<Evaluation>

1. Particle Diameters of Natural-Ceramide-Containing Particles

Immediately after the preparation, the particle diameter of the natural-ceramide-containing particles (or oil droplet-like dispersion particles containing thereof) in the ceramide dispersion were measured using a dynamic light scattering particle diameter distribution measuring device HLB-550 (Horiba, Ltd.). The measurement of the particle diameters was performed using a quartz cell after the natural-ceramide-containing particles were diluted with pure water to have the concentration of 1% by mass. The particle diameter was obtained as a median diameter when the refractive index of the sample, the refractive index of the dispersion medium, and the viscosity of the dispersion medium were set to 1.600, 1.333 (pure water), and the viscosity of pure water, respectively.

2. Evaluation of Stability over Time of Ceramide Dispersions

The stability over time was evaluated in the following manner using turbidity.
The turbidity of the ceramide dispersions 1 to the ceramide dispersion 13 of the examples and the comparative examples was measured using the UV-VIBLE SPECTRUM PHOTOMETER UV-2550 (trade name, manufactured by Shimadze Corporation) at the absorbance of 660 nm in a 10 mm cell (measurement temperature: temperature 25°C).
Furthermore, each ceramide dispersion was placed in a constant temperature reservoir at 60°C for 24 hours and then in a refrigerator at 4 °C for 24 hours back and forth 7 times (for two weeks), and then placed back at 25°C. Then, the turbidity was measured again, and evaluation was performed based on the criteria below by comparing the turbidity difference between before and after the preparation. The results are shown in Table 3 below.

D: Turbidity variation of 0.1 or more (not permissible from the viewpoints of product value)
C: Turbidity variation of from 0.05 to less than 0.1 (somewhat permissible from the viewpoints of product value)
B: Turbidity variation of from 0.01 to less than 0.05 (the variation is detectable, but presents no problem from the viewpoints of product value)
A: Turbidity variation of less than 0.01 (difficult to visually observe the variation)

[TABLE 1]

| | Ceramide dispersion liquid | Natural ceramide-containing particles | | State immediately after dispersion | Aging stability | | |
|---|---|---|---|---|---|---|---|
| | | Content of natural ceramides with respect to total mass of oil component (mass %) | Average diameter (nm) | | Turbidity variation | Evaluation | State observation |
| Example 1 | Ceramide dispersion liquid 1 | 100 | 1.8 | Transparent | 0.005 | A | difficult to observe variation |
| Example 2 | Ceramide dispersion liquid 2 | 100 | 1.5 | Transparent | 0.005 | A | difficult to observe variation |
| Example 3 | Ceramide dispersion liquid 3 | 100 | 30.7 | Transparent | 0.025 | B | Transparent, but slight variation in transparency |
| Example 4 | Ceramide dispersion liquid 4 | 100 | 19.5 | Transparent | 0.013 | B | Transparent, but slight variation in transparency |
| Example 5 | Ceramide dispersion liquid 5 | 100 | 38.3 | Transparent | 0.045 | B | Transparent, but slight variation in transparency |
| Example 6 | Ceramide dispersion liquid 6 | 50 | 58.3 | White transparent | 0.013 | B | Transparent, but slight variation in transparency |
| Examples 7 | Ceramide dispersion liquid 7 | 80 | 28.3 | Transparent | 0.017 | B | Transparent, but slight variation in transparency |
| Example 8 | Ceramide dispersion liquid 8 | 100 | 1.6 | Transparent | 0.003 | A | difficult to observe variation |
| Comparative Example 1 | Ceramide dispersion liquid 9 | 100 | 143 | White turbid | 0.289 | D | White turbid, not transparent |
| Comparative Example 2 | Ceramide dispersion liquid 10 | 100 | 122 | White Transparent | 0.083 | C | Precipitate |

(continued)

| | Ceramide dispersion liquid | Natural ceramide-containing particles | | State immediately after dispersion | Aging stability | | |
|---|---|---|---|---|---|---|---|
| | | Content of natural ceramides with respect to total mass of oil component (mass %) | Average diameter (nm) | | Turbidity variation | Evaluation | State observation |
| Comparative Example 3 | Ceramide dispersion liquid 11 | 40 | 205 | White turbid | 0.076 | D | Precipitate, White turbid, not transparent |
| Comparative Example 4 | Ceramide dispersion liquid 12 | 80 | 56.5 | White turbid | 0.162 | D | White turbid, not transparent |
| Comparative Example 5 | Ceramide dispersion liquid 13 | 100 | Not measurable due to white turbidity | White turbid | Not measurable | D | Measurement stopped |

As shown clearly in Table 3, it was found that the ceramide dispersion according to the present invention has small particle diameters of the natural-ceramide-containing particles contained therein and is also excellent in terms of the stability over time.

BRIEF DESCRIPTION OF DRAWINGS

Fig.1 is an exploded perspective view of a micro device as an example of a micro mixer.
Fig. 2 is a pattern cross-sectional view of a T-shape micro reactor showing an example of the mixing mechanism of a T-shape micro reactor.
Fig. 3 is a conceptual view of a T-shape micro reactor showing an example of the mixing mechanism of a T-shape micro reactor.

DESCRIPTION OF SYMBOL

100 MICRODEVICE
102 SUPPLY ELEMENT
104 CONFLUENCE ELEMENT
106 DISCHARGE ELEMENT
124 MICROCHANNEL
126 MICROCHANNEL
128 CENTER

**Claims**

**1.** A ceramide dispersion comprising at least:

natural-ceramide-containing particles that are dispersed in an aqueous phase as an oil-phase component; and at least one surfactant,
wherein
the natural-ceramide-containing particles contain one or more natural ceramides in an amount of 50% by mass or higher with respect to the total mass of oil components contained in an oil phase, and have a volume average particle diameter of from 0.5 nm to 100 nm; and
the ceramide dispersion contains, in the at least one surfactant, at least one polyglycerin fatty acid ester having an HLB of from 10 to 16.

**2.** The ceramide dispersion according to Claim I, wherein the at least one surfactant is contained in a range of from 0.1 parts by mass to 2 parts by mass with respect to the total mass of the one or more natural ceramides.

**3.** The ceramide dispersion according to Claim 1 or 2, wherein the polyglycerin fatty acid ester having an HLB of from 10 to 16 is a decaglycerin fatty acid ester.

**4.** The ceramide dispersion according to Claim 3, wherein the decaglycerin fatty acid ester is decaglycerin oleate.

**5.** The ceramide dispersion according to Claim 1 or 2, containing, in the at least one surfactant, decaglycerin oleate and a polyglycerin fatty acid ester having a polymerization degree of glycerin of less than 10 and a carbon number of the fatty acid of from 12 to 18.

**6.** The ceramide dispersion according to Claim 5, wherein the polyglycerin fatty acid ester having a polymerization degree of the glycerin of less than 10 and a carbon number of the fatty acid of from 12 to 18 is at least one selected from a hexaglycerin fatty acid ester or a tetraglycerin fatty acid ester and has an HLB of from 5.0 to 15.

**7.** The ceramide dispersion according to any one of Claims 1 to 6, wherein the one or more natural ceramides do not include sphingoglycolipid and each have three or more hydroxyl groups in a molecular structure thereof.

**8.** A cosmetic material comprising the ceramide dispersion according to any one of Claims 1 to 7.

**9.** A food product comprising the ceramide dispersion according to any one of Claims 1 to 7.

**10.** A pharmaceutical product comprising the ceramide dispersion according to any one of Claims 1 to 7.

FIG.1

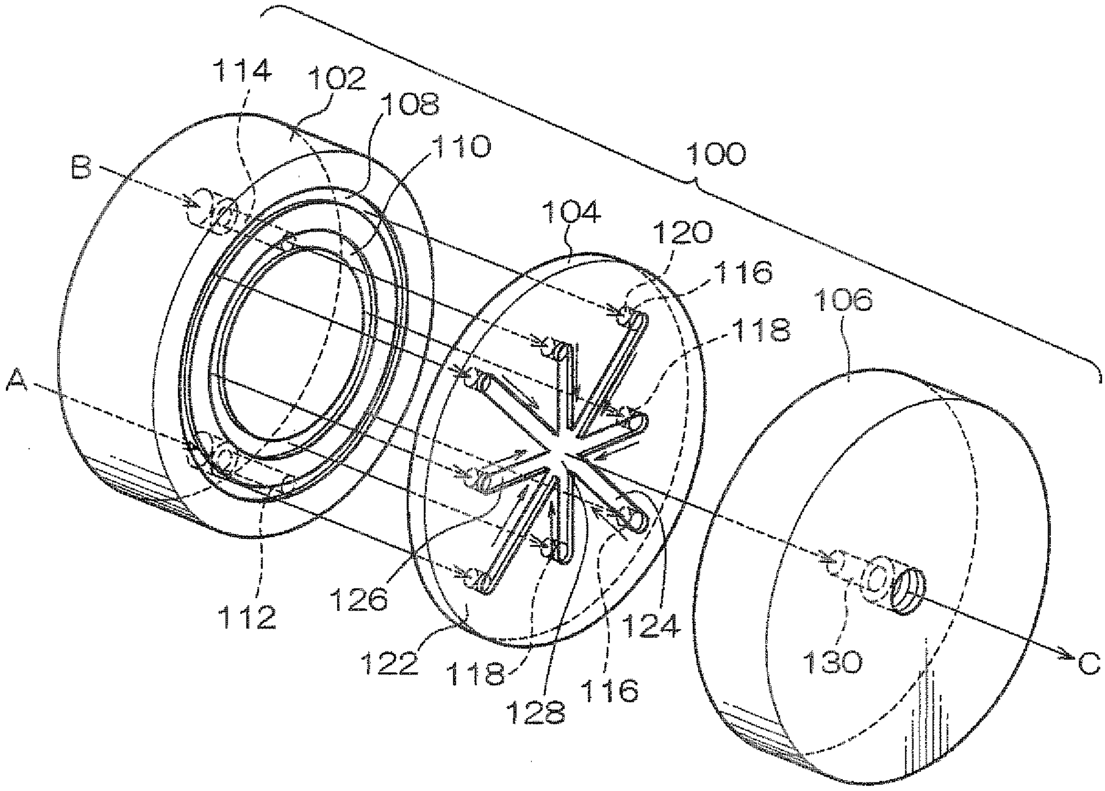
100
102
104
106
108
110
112
114
116
118
120
122
124
126
128
130
A
B
C

FIG.2

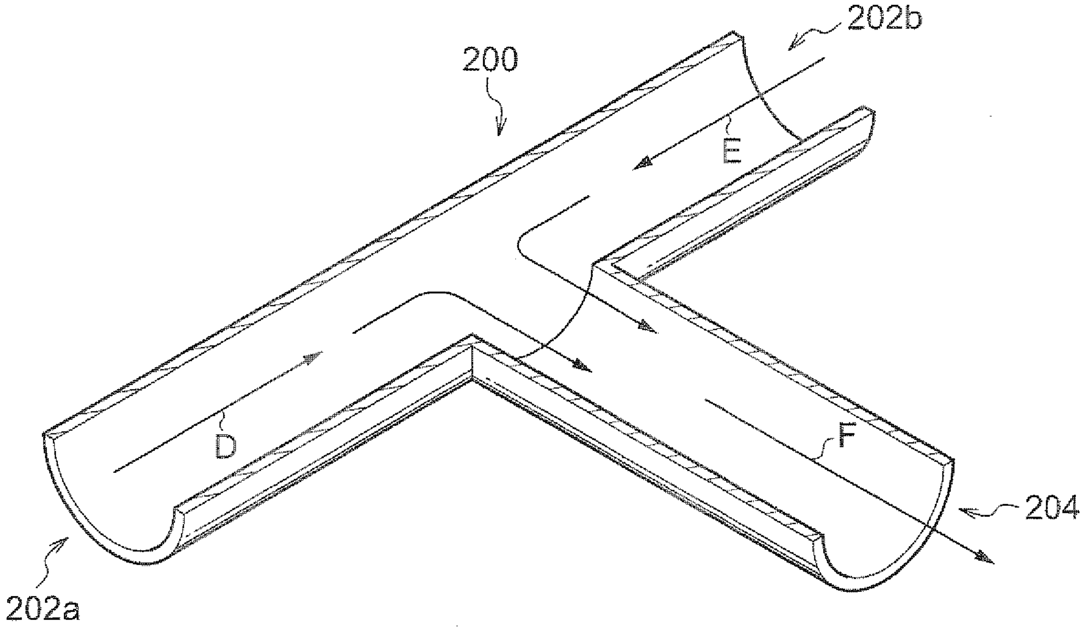
200
202a
202b
204
D
E
F

FIG.3

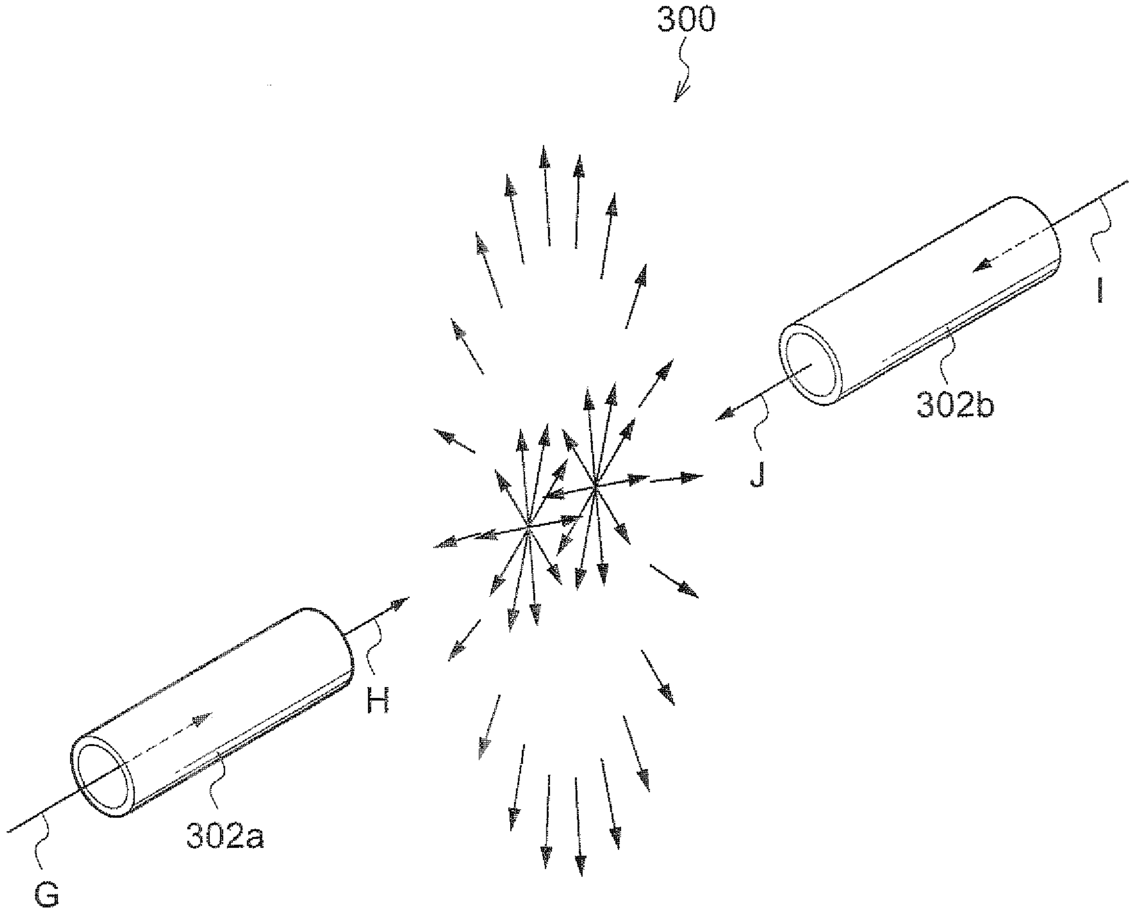

**INTERNATIONAL SEARCH REPORT**

International application No.
PCT/JP2009/059865

A. CLASSIFICATION OF SUBJECT MATTER
A61K8/68(2006.01)i, A23L1/30(2006.01)i, A61K8/06(2006.01)i, A61K8/86(2006.01)i, A61K31/164(2006.01)i, A61Q19/00(2006.01)i, B01J13/00(2006.01)i, A61P17/16(2006.01)n

According to International Patent Classification (IPC) or to both national classification and IPC

B. FIELDS SEARCHED

Minimum documentation searched (classification system followed by classification symbols)
A61K8/68, A23L1/30, A61K8/06, A61K8/86, A61K31/164, A61Q19/00, B01J13/00, A61P17/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2003-113393 A (NOF Corp.), 18 April, 2003 (18.04.03), Particularly, example 1 (Family: none) | 1-10 |
| A | JP 8-256729 A (NOF Corp.), 08 October, 1996 (08.10.96), Full text (Family: none) | 1-10 |
| A | JP 2002-114631 A (Kao Corp.), 16 April, 2002 (16.04.02), Full text (Family: none) | 1-10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>25 August, 2009 (25.08.09) | Date of mailing of the international search report<br>08 September, 2009 (08.09.09) |
| Name and mailing address of the ISA/<br>Japanese Patent Office<br>Facsimile No. | Authorized officer<br><br>Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000051676 A
- JP 7187987 A
- JP 2006335692 A
- JP 11310512 A
- JP 2001139796 A
- JP 2001316217 A
- JP 2049091 A
- JP 2005288254 A
- JP 2004033901 A
- JP 2008141181 A


### Non-patent literature cited in the description


- *Astaxanthin-no-kagaku,* 2005
- **H. Nagasawa et al.** *Chem. Eng. Technol.,* 2005, vol. 28 (3), 324-330